# EUROPEAN PATENT SPECIFICATION

(11) **EP 3 793 999 B1**
(45) Date of publication and mention of the grant of the patent: **15.01.2025**
(21) Application number: 19804097.4
(22) Date of filing: 17.05.2019
(51) Int. Cl.: C07D 471/04, C07D 231/14, C07D 213/81, C07D 213/82, C07D 239/28, C07D 239/34, C07D 241/24, C07D 249/10, C07D 271/10, C07D 277/56, A61P 29/00, A61K 31/4245, A61K 31/44, A61K 31/4412, A61K 31/4965, A61K 31/519

(54) **PAIN TREATING COMPOUNDS AND USES THEREOF**
SCHMERZBEHANDLUNGSVERBINDUNGEN UND DEREN VERWENDUNGEN
COMPOSÉS DE TRAITEMENT DE LA DOULEUR ET LEURS UTILISATIONS

(30) Priority: 17.05.2018 AU 2018901724; 20.08.2018 AU 2018903046
(43) Date of publication of application: 24.03.2021
(73) Proprietor: Bionomics Limited, Eastwood SA 5063 (AU)
(72) Inventor: HUFF, Belinda, Eastwood SA 5063 (AU); HOLLIS, Courtney, Mitchell Park South Australia 5043 (AU); TOOP, Hamish, Eastwood SA 5063 (AU); KUCHEL, Nathan, Eastwood SA 5063 (AU); MITCHELL, Lorna Helen, Eastwood SA 5063 (AU); SINGH, Rajinder, Torrensville South Australia 5031 (AU); AVERY, Thomas, Eastwood SA 5063 (AU); MICHAUT-SIMON, Celine, 67400 Illkirch (FR); CONTRERAS, Jean-Marie, 67230 Westhouse (FR); MORICE, Christophe, 68320 Widensolen (FR)
(74) Representative: Vossius & Partner Patentanwälte Rechtsanwälte mbB
(86) International application number: PCT/AU2019/050471
(87) International publication number: WO 2019/218024

(56) References cited:
- EP-A1- 1 669 348
- EP-A1- 1 782 811
- EP-A2- 0 279 681
- WO-A1-2013/131018
- GB-A- 2 448 056
- US-A1- 2011 009 454
- LUNNEY ET AL.: "Structure-based design of a novel series of nonpeptide ligands that bind to the pp60src SH2 domain", J. AM. CHEM. SOC., vol. 119, no. 51, 24 December 1997 (1997-12-24), pages 12471 - 12476, XP002095200, ISSN: 0002-7863, DOI: 10.1021/JA971794T
- DATABASE CAS 9 April 2018 (2018-04-09), "5- Azaspiro[2.3]hexane-5-carboxamide, 1,1 -difluoro-N - [ [ 4-[(2- fluorophenyl)methoxy]phenyl]methyl", XP55844418, retrieved from STN Database accession no. RN-2208879-86-9
- DATABASE CAS 23 March 2013 (2013-03-23), "Benzamide, N- [[ 4-[(2-fluorophenyl)methoxy ]phenyl]methyl]-4-[(1-oxo-2-propen-1-yl)amino", XP55844422, retrieved from STN Database accession no. RN-2197985-84-3
- DATABASE CAS 22 March 2018 (2018-03-22), "1H-1,4- Diazepine-1-carboxamide, N-[[4-[(2-fluorophenyl)methoxy]phenyl]methyl]hexahydro- 4-methyl-5-oxo", XP55844512, retrieved from STN Database accession no. RN-2196704-24-0
- DATABASE REGISTRY ANONYMOUS: "3-Isoxazolecarboxamide, 5-(4-chlorophenyl)-N-[[4-[(2- fluorophenyl)methoxy]phenyl]methyl]", XP055845492, retrieved from STN Database accession no. RN-2194450-94-5
- DATABASE REGISTRY ANONYMOUS: "2-Azabicyclo[2.2.2]octane-2-carboxamide, N-[[4-[(2-fluorophenyl)methoxy]phenyl]methyl]-6-(hydroxymethyl)", XP055845499, retrieved from STN Database accession no. 2193367-81-4
- DATABASE Registry ANONYMOUS -A: "4-Quinolinecarboxamide, N-[[4-[(2-fluorophenyl)methoxy]phenyl]methyl]-2- phenyl", XP055845502, retrieved from STN Database accession no. 2175518-51-9
- DATABASE REGISTRY ANONYMOUS: "3-Piperidinecarboxylic acid, 1-[[[[4-[(2- fluorophenyl)methoxy]phenyl]methyl]amino]carbonyl]", XP055845507, retrieved from STN Database accession no. 2040529-30-2
- DATABASE REGISTRY ANONYMOUS: "3-Pyrrolidinecarboxylic acid, 1-[[[[4-[(2- fluorophenyl)methoxy]phenyl]methyl]amino]carbonyl]", XP055845517, retrieved from STN Database accession no. RN-2035593-89-4
- DATABASE REGISTRY ANONYMOUS: "4-Piperidinecarboxylic acid, 1-[[[[4-[(2- fluorophenyl)methoxy]phenyl]methyl]amino]carbonyl]-", XP055845524, retrieved from STN Database accession no. 1940975-22-3
- DATABASE REGISTRY ANONYMOUS: "3-Pyrrolidinecarboxylic acid, 1-[[[[4-[(2- fluorophenyl)methoxy]phenyl]methyl]amino]carbonyl]-3-methyl", XP055845533, retrieved from STN Database accession no. RN-1940126-25-9
- DATABASE REGISTRY ANONYMOUS: "1,4-Dioxin-2-carboxamide, N-[[4-[(2-fluorophenyl)methoxy]phenyl]methyl]- 5,6-dihydro", XP055845535, retrieved from STN Database accession no. 1935809-78-1
- DATABASE REGISTRY ANONYMOUS: "2-Pyrrolidinecarboxamide, N-[[4-[(2-fluorophenyl)methoxy]phenyl]methyl]-5- oxo", XP055845540, retrieved from STN Database accession no. RN-1935693-34-7
- DATABASE REGISTRY ANONYMOUS: "-4-Thiazolidinecarboxamide, N-[[4-(cyclopentylmethoxy)phenyl]methyl]-2-(3- pyridinyl)", XP055845544, retrieved from STN Database accession no. 735232-95-8

## Description

### FIELD

The present invention relates to compounds useful in the modulation of ion channel activity in cells. The invention also relates to these compounds for use in the treatment of pain, and pharmaceutical compositions containing these compounds, and discloses methods for their preparation.

### BACKGROUND

Voltage gated sodium channels are essential for the initiation and propagation of action potentials in excitable tissues such as muscle and nerve. Sodium channels are expressed principally in the central nervous system (CNS) and peripheral nervous system (PNS) on neurons and glial cells. To date nine subtypes have been identified: Nav1.1, Nav1.2, Nav1.3 and Nav1.6 which occur predominantly in the CNS (and less in the PNS), Nav1.4 which is specific to skeletal muscle, Nav1.5 in cardiac muscle and Nav1.7-1.9 which are distributed primarily in the peripheral nervous system in sensory neurons. Increased Nav1.x channel activity leads to nerve hyper excitability and underlies a number of pathological conditions including: epilepsy (Nav1.1 and Nav1.2), Cardiac arrhythmia (Nav1.5), myotonia (Nav1.4) and pain (Nav1.3, Nav1.7, Nav1.8 and Nav 1.9).

Pharmacological, molecular and genetic studies in both human and rodents, have identified several channels as pivotal for pain signal transmission, including Nav1.3, Nav1.7, Nav1.8, and Nav1.9. Genetic mutations in the SCN9A gene, resulting in a loss of function mutation in Nav1.7 lead to congenital insensitivity to pain (Cox et al, Nature. (2006) 444(7121) 894-898; Goldberg et al., 2007), while gain-of-function mutations cause debilitating pain syndromes such as erythromelalgia, paroxysmal extreme pain disorder, and small-fiber neuropathy (Yang et al., 2004; Fertleman et al., 2006; Faber et al., 2012). Furthermore, genetic linkages studies have shown mutations in the SCN10A gene which encodes Nav1.8 results in hyperexcitability and decreased pain thresholds in small fiber neuropathy patients, and SCN11A (Nav1.9) mutations, that produce gain-of-function effects in painful peripheral neuropathy and familial episodic pain.

US2011/009454 A1 and GB 2448056A relate to an agricultural composition for controlling or preventing plant diseases caused by plant pathogenic microbes, which comprises specific amide compounds, salts thereof or hydrates thereof.

Lunney et al, describes a structure-based design of a novel series of nonpeptide ligands that bind to the pp60src SH2 domain (J.AM.CHEM.SOC. 1997, vol 119, no. 51, pp 12741-12476).

WO 2013/131018 A1 relates to certain biaryl derivatives reported to be useful in treating conditions associated with voltage-gated ion channel function.

EP 1782811 A1 discloses specific heterocyclic compounds as novel antimalarial agents.

EP 1669348 A1 describes certain heterocyclic compounds as novel antifungal agents.

EP0279681 A2 discloses saturated heterocyclic carboxamide derivatives having a platelet activating factor (PAF) antagonizing (anti-PAF) activity.

Further, search log file Registry of 23 Sep 2021, hits 1/22-15/22 and 21/22-22/22, discloses compounds similar to the compounds of the present invention.

Sodium channel blockers are commonly used as analgesics and are represented across three drug classes: local anaesthetics, class I antiarrhythmic and antiepileptic drugs. Although generally well tolerated, these drugs exhibit poor Nav1.x subtype selectivity and have a limited dose range due to side effects expected of interfering with CNS, cardiac and skeletal muscle sodium channel function including convulsions, ataxia, motor impairment, arrhythmias and paralysis. Despite their poor Nav1.x selectivity, these drugs are nevertheless tolerated due to their relatively low potency for Nav1.x channels in the resting (closed) state and greater potency for the inactivated state which commonly presides in Nav1.x channels mediating pain signals. In addition to this "state-dependence", the potency of local anaesthetic like compounds tends to increase with the frequency of channel opening (use-dependence) which again presides more in pathogenic states such as pain, epilepsy and ventricular fibrillation. Thus Nav1.x blockers can exhibit functional selectivity as well as subtype selectivity.

Pain has both peripheral and central components that are affected by the large network of pathways, thus peripheral neuron-targeted drugs may need to be BBB permeant to affect their actions. Nav1.7 is distributed primarily in the peripheral nervous system in dorsal root and sympathetic ganglia where it plays a role in setting the threshold for action potential firing thus controlling sensory neuron sensitivity to incoming stimuli. However, peripheral sensory neurons have terminals in the spinal cord within the blood brain barrier (BBB) and these terminals have high concentrations of Nav1.7 channels that is involved in neurotransmitter release. Hence, although Nav1.7 is distributed primarily in the peripheral nervous system, Nav1.7 inhibitors may need to be CNS penetrant, to block all aspects of Nav1.7 function.

Development of a potent and subtype selective inhibitors for any one or more of Nav1.7, Nav 1.3, Nav1.8 and Nav 1.9 are expected to provide substantial benefit over existing analgesics targeting sodium channels which lack selectivity and consequently are associated with a range of dose limiting CNS and cardiovascular side effects.

There is still a need for improved and specific therapies for the treatment of pain.

### SUMMARY OF THE INVENTION

The present invention provides compounds of formula (I) as defined in the appended claims and pharmaceutical compositions thereof. In certain embodiments compounds of formula (I) have utility in the treatment of pain disorders. Specifically, the present invention relates to:
A compound of the formula (I) 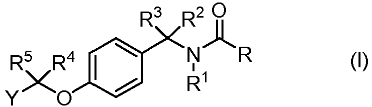
or salts, stereoisomers, or solvates thereof,
wherein
R is optionally substituted 7-12 membered heteroaryl with 2 or more N atoms;
R¹ is H or C₁-C₄ alkyl;
R², R³, R⁴ and R⁵ are independently H or C₁-C₄ alkyl, or R² and R³, or R⁴ and R⁵ together form a cycloalkyl ring;
Y is selected from 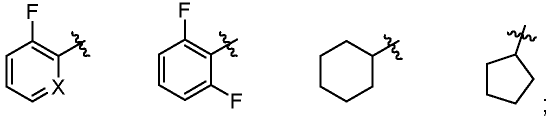 and
X is CH or N,
wherein the optional substituents are selected from the group consisting of halogen, C₁₋₆ alkyl, C₁₋₆ alkoxy, C₂₋₆ alkenyl, C₂₋₆ alkynyl, C₁₋₆ haloalkyl, C₁₋₆ haloalkoxy, C₁₋₆ alkoxy(C₁₋₆)alkyl, C₃₋₆ cycloalkyl, -OH, phenyl, benzyl, phenoxy, benzyloxy, benzoyl, - NH₂, -NHC₁₋₄ alkyl, -N(C₁₋₄ alkyl)₂, -CN, -NO₂, mercapto, -P=O(OH)(NH₂), -S(O)₂NH₂, -S(O)₂NHC₁₋₄ alkyl, -S(O)₂N(C₁₋₄ alkyl)₂, C₁₋₆ alkylcarbonyl, C₁₋₆ alkoxycarbonyl, CO₂H, -S(O)R‴ (where R'" is C₁₋₆ alkyl or cycloalkyl) and -S(O)₂R‴ (where R'" is C₁₋₆ alkyl, cycloalkyl or OH), and
wherein the "cycloalkyl ring" is a ring selected from cyclopropyl, cyclobutyl, cyclopentyl and cyclohexyl.

In a further aspect, the present invention relates to a pharmaceutical composition comprising a compound of Formula (I) together with a diluent or carrier adjuvant.

The present invention also provides a compound of formula (I) 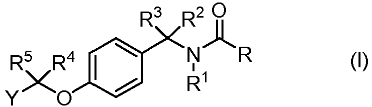
or salts, stereoisomers, or solvates thereof, for use in treating or preventing pain disorders,
wherein
R is optionally substituted aryl, optionally substituted heterocycyl or optionally substituted heteroaryl;
R¹ is H or optionally substituted C₁-C₄ alkyl;
R², R³, R⁴ and R⁵ are independently H or C₁-C₄ alkyl or R² and R³, or R⁴ and R⁵ together form a cycloalkyl ring;
Y is selected from 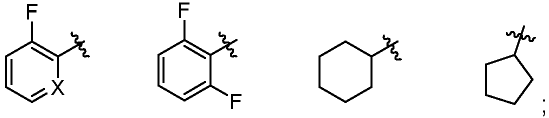 and
X is CH or N,
wherein the optional substituents are selected from the group consisting of halogen, C₁₋₆ alkyl, C₁₋₆ alkoxy, C₂₋₆ alkenyl, C₂₋₆ alkynyl, C₁₋₆ haloalkyl, C₁₋₆ haloalkoxy, C₁₋₆ alkoxy(C₁₋₆)alkyl, C₃₋₆ cycloalkyl, -OH, phenyl, benzyl, phenoxy, benzyloxy, benzoyl, - NH₂, -NHC₁₋₄ alkyl, -N(C₁₋₄ alkyl)₂, -CN, -NO₂, mercapto, -P=O(OH)(NH₂), -S(O)₂NH₂, -S(O)₂NHC₁₋₄ alkyl, -S(O)₂N(C₁₋₄ alkyl)₂, C₁₋₆ alkylcarbonyl, C₁₋₆ alkoxycarbonyl, CO₂H, -S(O)R‴ (where R'" is C₁₋₆ alkyl or cycloalkyl) and -S(O)₂R‴ (where R'" is C₁₋₆ alkyl, cycloalkyl or OH), and
wherein the "cycloalkyl ring" is a ring selected from cyclopropyl, cyclobutyl, cyclopentyl and cyclohexyl;
"aryl" is selected from phenyl, biphenyl, napthyl, tetrahydronaphthyl, indenyl, azulenyl, fluorenyl and anthracenyl;
"heterocyclyl" is a monovalent saturated or unsaturated group with 1 to 8 carbon atoms and from 1 to 4 heteroatoms selected from N, S and O; and
"heteroaryl" is a monovalent aromatic carbocyclic group with 2 to 10 carbon atoms and 1 to 4 heteroatoms selected from O, N and S.

Herein disclosed are further:
Compounds of formula (I) 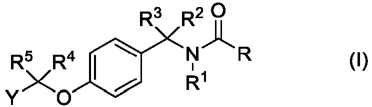
or salts, stereoisomers, solvates or prodrugs thereof,
wherein
R is optionally substituted aryl, optionally substituted heterocycyl or optionally substituted heteroaryl;
R¹ is H or optionally substituted C₁-C₄ alkyl;
R², R³, R⁴ and R⁵ are independently H or C₁-C₄ alkyl or R² and R³, or R⁴ and R⁵ together form a cycloalkyl ring;
Y is selected from 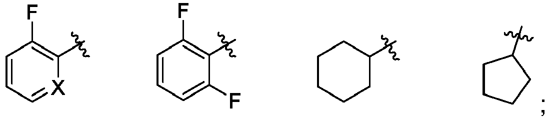 and
X is CH or N.

Compounds of the formula (I) 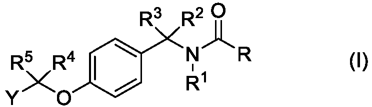
or salts, stereoisomers, solvates or prodrugs thereof,
wherein
R is optionally substituted heteroaryl;
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 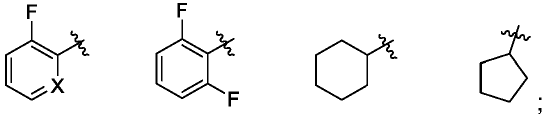 and
X is CH or N.

Compounds of the formula (I) 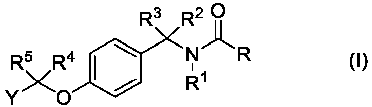
or salts, stereoisomers, solvates or prodrugs thereof,
wherein
R is optionally substituted 7-12 membered heteroaryl;
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 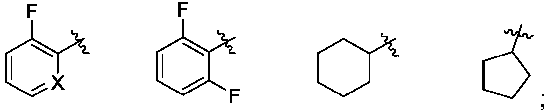 and
X is CH or N.

Compounds of the formula (I) 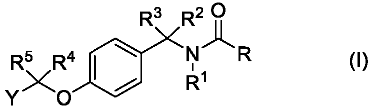
or salts, stereoisomers, solvates or prodrugs thereof,
wherein
R is optionally substituted 7-12 membered heteroaryl with 2 or more N atoms;
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 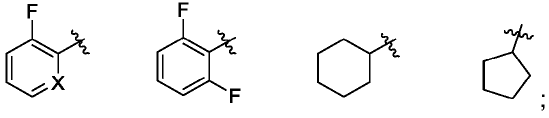 and
X is CH or N.

Compounds of the formula (I) 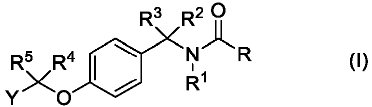
or salts, stereoisomers, solvates or prodrugs thereof,
wherein
R is optionally substituted 7-12 membered heteroaryl with 2 nitrogen atoms;
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 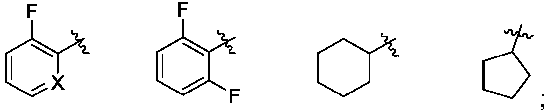 and
X is CH or N.

In another aspect, the present invention relates to pharmaceutical compositions comprising of at least one compound provided herein and a pharmaceutical carrier, excipient or diluent. The pharmaceutical composition can comprise one or more compounds described herein. It will be understood that compounds provided herein useful in the pharmaceutical composition and treatment methods disclosed below, can be pharmaceutically acceptable as prepared and used.

In still a further aspect, the invention provides or relates to the claimed compounds for use in methods for preventing, treating or ameliorating a condition from among those listed herein, particularly pain disorders (including pain associated with cancer, surgery, and bone fracture, acute pain, inflammatory pain and neuropathic pain). Pain disorders for which the compounds of the invention may be useful include neuropathic pain (such as postherpetic neuralgia, nerve injury, the "dynias", e.g., vulvodynia, phantom limb pain, root avulsions, painful diabetic neuropathy, painful traumatic mononeuropathy, painful polyneuropathy); central pain syndromes (potentially caused by virtually any lesion at any level of the nervous system); postsurgical pain syndromes (eg, postmastectomy syndrome, postthoracotomy syndrome, stump pain); bone and joint pain (osteoarthritis), repetitive motion pain, dental pain (e.g., toothache), cancer pain, myofascial pain (muscular injury, fibromyalgia); perioperative pain (general surgery, gynecological), chronic pain, dysmennorhea, as well as pain associated with angina, and inflammatory pain of varied origins (e.g. osteoarthritis, rheumatoid arthritis, rheumatic disease, teno- synovitis and gout), headache, migraine and cluster headache, headache, primary hyperalgesia, secondary hyperalgesia, primary allodynia, secondary allodynia, or other pain caused by central sensitization.

In an embodiment the invention relates to the claimed compounds for use in the treatment of chronic pain.

In an embodiment the invention relates to the claimed compounds for use in the treatment of neuropathic pain.

In an embodiment the invention relates to the claimed compounds for use in the treatment of inflammatory pain.

In an embodiment the invention relates to the claimed compounds for use in the treatment of cancer pain.

In an embodiment the invention relates to the claimed compounds for use in the treatment of trigeminal neuralgia, lower back pain, post- operative pain, toothache, arthritic pain (rheumatoid osteoarthritis, gout), pain from irritable bowel, inherited erythromelalgia, paroxysmal extreme pain syndrome, post herpetic neuralgia (shingles), musculoskeletal pain, multiple sclerosis, sciatica, diabetic neuropathy, and HIV related neuropathy.

In an embodiment the invention relates to the claimed compounds for use in the treatment of chronic itch.

In an embodiment the invention relates to the claimed compounds for use in the treatment of pathological cough.

In a further embodiment the invention provides compounds to treat or prevent conditions resulting from membrane hyperexcitablility mediated by aberrant Nav channel activity for state and use- dependent Nav blockers; including:
CNS conditions (for instance, epilepsy, anxiety, depression, bipolar);
Cardiac conditions (for instance, arrhythmias, atrial and ventricular fibrillation); and Muscular (for instance, restless leg, tetanus).

In addition to methods of treatment set forth above, the present invention extends to the use of any of the compounds of the invention in the preparation of medicaments that may be administered for such treatments, as well as to such compounds for the treatments disclosed and specified.

Herein disclosed are also methods for synthesising the compounds described herein, with representative synthetic protocols and pathways described below.

Accordingly, in certain embodiments it is an aim of the present invention to provide new compounds that can modulate the activity of at least one voltage-gated sodium ion channel and thus prevent or treat any conditions that may be casually related to aberrations in such acts.

Accordingly the invention provides compounds that can treat or alleviate maladies or symptoms of same, such as pain, that may be causally related to the activation of a sodium channel.

In an embodiment the compounds of the present invention display subtype and/or functional selectivity in relation to anyone one or more of Nav1.7, Nav1.3, Nav1.8 and Nav1.9 inhibition.

### DETAILED DESCRIPTION

The term "alkyl" as used alone or in combination herein refers to a straight or branched chain saturated hydrocarbon group. The term "C₁₋₁₂ alkyl" refers to such a group containing from one to twelve carbon atoms and "lower alkyl" refers to C₁₋₆ alkyl groups containing from one to six carbon atoms, such as methyl ("Me"), ethyl ("Et"), n-propyl, isopropyl, n-butyl, isobutyl, sec-butyl, tert-butyl and the like.

The term "cycloalkyl" refers to non-aromatic, saturated non-aromatic carbocycles. The term "C₃₋₉ cycloalkyl", for instance, refers to such a group having from 3 to 9 carbon atoms. Examples include cyclopropyl, cyclobutyl, cyclopentyl and cyclohexyl.

The term "alkenyl" refers to a straight or branched hydrocarbon containing one or more double bonds, preferably one or two double bonds. The term "C₂₋₁₂ alkenyl", for instance, refers to such a group containing from two to twelve carbon atoms. Examples of alkenyl include allyl, 1-methylvinyl, butenyl, iso-butenyl, 1,3-butadienyl, 3-methyl-2-butenyl, 1,3-butadienyl, 1,4-pentadienyl, 1-pentenyl, 1-hexenyl, 3-hexenyl, 1,3-hexadienyl, 1,4-hexadienyl and 1,3,5-hexatrienyl.

The term "cycloalkenyl" refers to cyclic alkenyl groups having a single cyclic ring or multiple condensed rings, and at least one point of internal unsaturation, preferably incorporating 4 to 11 carbon atoms. Examples of suitable cycloalkenyl groups include, for instance, cyclobut-2-enyl, cyclopent-3-enyl, cyclohex-4-enyl, cyclooct-3-enyl, indenyl and the like.

The term "alkynyl" refers to a straight or branched hydrocarbon containing one or more triple bonds, preferably one or two triple bonds. The term "C₂₋₁₂ alkynyl", for instance, refers to such a group containing from two to twelve carbon atoms. Examples include 2-propynyl and 2- or 3-butynyl.

The term "alkoxy" as used alone or in combination refers to a straight or branched chain alkyl group covalently bound via an oxygen linkage (-O-) and the terms "C₁₋₆ alkoxy" and "lower alkoxy" refer to such groups containing from one to six carbon atoms, such as methoxy, ethoxy, propoxy, isopropoxy, butoxy, t-butoxy and the like.

The term "aryl" refers to carbocyclic (non-heterocyclic) aromatic rings or ring systems. The aromatic rings may be mono- or bi-cyclic ring systems. The aromatic rings or ring systems are generally composed of 5 to 10 carbon atoms. Examples of suitable aryl groups include but are not limited to phenyl, biphenyl, naphthyl, tetrahydronaphthyl, and the like.

Aryl groups include phenyl, naphthyl, indenyl, azulenyl, fluorenyl or anthracenyl.

The term "heteroaryl" refers to a monovalent aromatic carbocyclic group, preferably of from 2 to 10 carbon atoms and 1 to 4 heteroatoms selected from oxygen, nitrogen and sulfur within the ring. Preferably the heteroatom is nitrogen. Such heteroaryl groups can have a single ring (e.g., pyridyl, pyrrolyl or furyl) or multiple condensed rings (e.g., indolizinyl, benzothienyl, or benzofuranyl).

The term "heterocyclyl" refers to a monovalent saturated or unsaturated group having a single ring or multiple condensed rings, preferably from 1 to 8 carbon atoms and from 1 to 4 hetero atoms selected from nitrogen, sulfur, oxygen, selenium or phosphorous within the ring.

Examples of 5-membered monocyclic heterocyclyl and heteroaryl groups include furyl, thienyl, pyrrolyl, H-pyrrolyl, pyrrolinyl, pyrrolidinyl, oxazolyl, oxadiazolyl, (including 1,2,3 and 1,2,4-oxadiazolyls) thiazolyl, isoxazolyl, furazanyl, isothiazolyl, pyrazolyl, pyrazolinyl, pyrazolidinyl, imidazolyl, imidazolinyl, triazolyl (including 1,2,3- and 1,3,4-triazolyls), tetrazolyl, thiadiazolyl (including 1,2,3- and 1,3,4-thiadiazolyls).

Examples of 6-membered monocyclic heterocyclyl and heteroaryl groups include pyridyl, pyrimidinyl, pyridazinyl, pyranyl, pyrazinyl, piperidinyl, 1,4-dioxanyl, morpholinyl, 1,4-dithianyl, thiomorpholinyl, piperazinyl, 1,3,5-trithianyl and triazinyl.

Examples of 8, 9 and 10-membered bicyclic heterocyclyl and heteroaryl groups include 1H thieno[2,3-c]pyrazolyl, thieno[2,3-b]furyl, indolyl, isoindolyl, benzofuranyl, benzothienyl, benzoxazolyl, benzothiazolyl, benzisoxazolyl, benzisothiazolyl, benzimidazolyl, indazolyl, isoquinolinyl, quinolinyl, quinoxalinyl, uridinyl, purinyl, cinnolinyl, phthalazinyl, quinazolinyl, quinoxalinyl, benzotriazinyl, naphthyridinyl, pteridinyl and the like.

The term "arylalkyl" refers to carbocyclic aromatic rings or ring systems as previously described and substituted by an alkyl group, also as previously described. Unless otherwise indicated the aryl substituent is attached by the alkyl part of the substituent. An example of an arylalkyl group is a benzyl group. Likewise the terms "aryl C₁₋₁₂ alkyl", "aryl C₂₋₁₂ alkenyl" and "aryl C₂₋₁₂ alkynyl" refer to carbocyclic aromatic rings or ring systems as previously described and substituted by a C₁₋₁₂ alkyl, C₂₋₁₂ alkenyl or C₂₋₁₂ alkynyl group, as previously described.

The terms "halo" and "halogen" refers to fluoro, chloro, bromo and iodo groups.

The term "halo alkyl" group has one or more of the hydrogen atoms on an alkyl group replaced with halogens. Notable examples are -CF₃ or -CF₂H.

The term "aryloxy" refers to an aryl group as earlier described linked to the parent structure via an oxygen linkage (-O-). A notable example is phenoxy. Similarly the term "heteroaryloxy" refers to a heteroaryl group as earlier described linked to the parent structure via an oxygen group. A notable example is a 4, 6 or 7-benzo[b]furanyloxy group.

The term "acyl" refers to groups H-C(O)-, alkyl-C(O)-, cycloalkyl-C(O)-, aryl-C(O)-, heteroaryl-C(O)- and heterocyclyl-C(O)-, where alkyl, cycloalkyl, aryl, heteroaryl and heterocyclyl are described herein.

The term "oxyacyl" refers to groups HOC(O)-, alkyl-OC(O)-, cycloalkyl-OC(O)-, aryl-OC(O)-, heteroaryl-OC(O)-, and heterocyclyl-OC(O)-, where alkyl, cycloalkyl, aryl, heteroaryl and heterocyclyl are as described herein.

The term "acylamino" refers to the group -NR"C(O)R" where each R" is independently hydrogen, alkyl, cycloalkyl, aryl, heteroaryl and heterocyclyl and where each of alkyl, cycloalkyl, aryl, heteroaryl and heterocyclyl are as described herein.

The term "aminoacylamino" refers to the group -NR"C(O)NR"R" where each R" is independently hydrogen, alkyl, cycloalkyl, aryl, heteroaryl and heterocyclyl and where each of alkyl, cycloalkyl, aryl, heteroaryl and heterocyclyl are as described herein.

The term "alkylene" refers to divalent alkyl groups preferably having from 1 to 10 carbon atoms and more preferably 1 to 6 carbon atoms. Examples of such alkylene groups include methylene (-CH₂-), ethylene (-CH₂CH₂-), and the propylene isomers (e.g., -CH₂CH₂CH₂- and -CH(CH₃)CH₂-), and the like.

The term "optionally substituted" means that a group may include one or more substituents. One or more hydrogen atoms on the group may be replaced by substituent groups independently selected from halogens (for example halo alkyl such as -CF₃ or -CF₂H), C₁₋₆ alkyl, C₂₋₆ alkenyl, C₂₋₆ alkynyl, -(CH₂)ₚC₃₋₇ cycloalkyl, -(CH₂)ₚC₄₋₇ cycloalkenyl, -(CH₂)ₚ aryl, -(CH₂)ₚ heterocyclyl, -(CH₂)ₚ heteroaryl, -C₆H₄S(O)_{q}C₁₋₆ alkyl, -C(Ph)₃, -CN, -OR, -O-(CH₂)₁₋₆-R, -O-(CH₂)₁₋₆-OR, - OC(O)R, -C(O)R, -C(O)OR, -OC(O)NR'R", - NR'R", -NRC(O)R', -NRC(O)NR'R", -NRC(S)NR'R", -NRS(O)₂R', -NRC(O)OR', -C(NR )NR'R", -C(=NOR')R, -C(=NOH)NR'R", -C(O)NR'R", -C(=NCN)-NR'R", -C(=NR)NR'R", -C(=NR')SR", -NR'C(=NCN)SR", -CONRSO₂R', -C(S)NR'R", - S(O)_{q}R, -SO₂NR'R", -SO₂NRC(O)R', -OS(O)₂R, -PO(OR)₂ and -NO₂;
where p is 0-6, q is 0-2 and each R, R' and R" is independently selected from H, C₁₋₆ alkyl, C₂₋₆ alkenyl, C₂₋₆ alkynyl, C₃₋₇ cycloalkyl, C₄₋₇ cycloalkenyl, aryl, heterocyclyl, heteroaryl, C₁₋₆ alkylaryl, C₁₋₆ alkylheteroaryl, and C₁₋₆ alkylheterocyclyl, wherein the alkyl, alkenyl, alkynyl, cycloalkyl, cycloalkenyl, aryl, heterocyclyl, heteroaryl, C₁₋₆ alkylaryl, C₁₋₆ alkylheteroaryl, or C₁₋₆ alkylheterocyclyl, may be optionally substituted with one to six of same or different groups selected from halogen, hydroxy, lower alkyl, lower alkoxy, -CO₂H, CF₃, CN, phenyl, NH₂ and -NO₂; or when R' and R" are attached to the same nitrogen atom, they may, together with the atom to which they are attached, form a 5 to 7 membered nitrogen containing heterocyclic ring.

A list of optional substituents includes: halogen (in particular, CI, Br or F), C₁₋₆ alkyl, C₁₋₆ alkoxy, C₂₋₆ alkenyl, C₂₋₆ alkynyl, C₁₋₆ haloalkyl (in particular -CF₃), C₁₋₆ haloalkoxy (such as -OCF₃), C₁₋₆ alkoxy(C₁₋₆)alkyl, C₃₋₆ cycloalkyl, -OH, phenyl, benzyl, phenoxy, benzyloxy, benzoyl, -NH₂, -NHC₁₋₄ alkyl, -N(C₁₋₄ alkyl)₂, -CN, -NO₂, mercapto, -P=O(OH)(NH₂), -S(O)₂NH₂, -S(O)₂NHC₁₋₄ alkyl, -S(O)₂N(C₁₋₄ alkyl)₂, C₁₋₆ alkylcarbonyl, C₁₋₆ alkoxycarbonyl, CO₂H, -S(O)R‴ (where R‴ is lower alkyl or cycloalkyl) and -S(O)₂R‴ (where R‴ is lower alkyl, cycloalkyl or OH).

Unless otherwise defined and only in respect of the ring atoms of non-aromatic carbocyclic or heterocyclic compounds, the ring atoms of such compounds may also be optionally substituted with one or two =O groups, instead of or in addition to the above described optional substituents.

When the optional substituent is or contains an alkyl, alkenyl, alkynyl, alkoxy, alkoxyalkyl, cycloalkyl, aryl, heteroaryl or heterocyclyl group, the group may itself be optionally substituted with one to six of the same or different substituents selected from halogen, C₁₋₆ alkyl, C₁₋₆ alkoxy, C₂₋₆ alkenyl, C₂₋₆ alkynyl, C₁₋₆ haloalkyl (in particular -CF₃), C₁₋₆ haloalkoxy (such as -OCF₃), -OH, phenyl, benzyl, phenoxy, benzyloxy, benzoyl, -NH₂, -NHC₁₋₄ alkyl, -N(C₁₋₄ alkyl)₂, -CN, -NO₂, mercapto, - P=O(OH)(NH₂), -S(O)₂NH₂, -S(O)₂NHC₁₋₄ alkyl, -S(O)₂N(C₁₋₄ alkyl)₂, C₁₋₆ alkylcarbonyl, C₁₋₆ alkoxycarbonyl, CO₂H, -S(O)R‴ (where R‴ is lower alkyl or cycloalkyl) and -S(O)₂R‴ (where R‴ is lower alkyl, cycloalkyl or OH).

In an embodiment, R is an optionally substituted aryl group. In another embodiment, R is an optionally substituted aryl group, wherein the aryl group is one or more fused 6-membered rings. In another embodiment, R is a monosubstituted aryl group. In another embodiment, R is an optionally substituted aryl group, wherein there is more than one substituent on the aryl group. In another embodiment, R is an unsubstituted phenyl group. In another embodiment, R is an optionally substituted phenyl group. In another embodiment, R is an optionally substituted phenyl group, wherein the substituents are selected from H, halogen, alkyl, alkenyl, alkynyl, acyl, amino, acylamino, nitro, nitrile, silyl, aryl and heteroaryl. In another embodiment, R is an optionally substituted phenyl group, wherein the substituent is a fluoro group. In another embodiment, R is an optionally substituted phenyl group, wherein the substituent is in the *para* position. In another embodiment, R is an optionally substituted phenyl group, wherein the substituent is in the *meta* position. In another embodiment, R is an optionally substituted phenyl group, wherein the substituent is in the *ortho* position. In a further embodiment, R is a para-fluoro-substituted phenyl group.

In an embodiment, R is an optionally substituted heterocycyl group. In another embodiment, R is an optionally substituted heterocycyl group, wherein the heterocycyl group is 5-12 membered. In another embodiment, R is an optionally substituted heterocycyl group, wherein the heterocycyl group is 6-12 membered. In another embodiment, R is an optionally substituted heterocycyl group, wherein the heterocycyl group is 6-10 membered. In another embodiment, R is an optionally substituted heterocycyl group, wherein the cycyl group is one or more fused rings. In another embodiment, R is an optionally substituted 5 membered heterocycyl group selected from, but not limited to, pyrrolidinyl, pyrazolidinyl, imidazolidinyl, tetrahydrofuranyl, 1,3-dioxolanyl, tetrahydrothiophenyl, 1,2-oxathiolanyl, 1,3-oxathiolanyl, sulfolanyl, 2,4-thiazolidinedionyl, succinimidyl, 2-oxazolidonyl, hydantoinyl and pyrrolidonyl. In another embodiment, R is an optionally substituted 6 membered heterocycyl group selected from, but not limited to, piperidinyl, piperazinyl, tetrahydropyranyl, 1,4-dioxanyl, thianyl, 1,3-dithianyl, 1,4-dithianyl, 1,3,5-trithianyl, morpholinyl and thiomorpholinyl. In another embodiment, R is an optionally substituted 7-12 membered heterocycyl group selected from, but not limited to, oxepanyl, azocanyl, thiocanyl and azonanyl. In another embodiment, R is an optionally substituted fused heterocycyl group selected from, but not limited to, pyrrolizidinyl, decahydroisoquinolinyl, decahydroquinolinyl, quinuclidinyl, 1-azaadamantanyl and 2-azaadamantanyl.

In an embodiment, R is an optionally substituted heteroaryl group. In another embodiment, R is an optionally substituted heteroaryl group, wherein the heteroaryl group is 5-membered or 6-membered. In another embodiment, R is an optionally substituted 5-membered heteroaryl group, selected from furan, pyrrole, thiophene, imidazole, pyrazole, oxazole, isoxazole and thiazole. In another embodiment, R is an optionally substituted 6-membered heteroaryl group selected from pyridine, pyrazine, pyrimidine, pyridazine and triazine. In another embodiment, R is an optionally substituted heteroaryl group, wherein the heteroaryl group is one or more fused 6-membered rings. In another embodiment, R is an optionally substituted heteroaryl group selected from quinolone, isoquinoline, quinoxaline and quinazoline. In another embodiment, R is an optionally substituted heteroaryl group, wherein the heteroaryl group has more than one heteroatom. In another embodiment, R is a monosubstituted heteroaryl group. In another embodiment, R is an optionally substituted heteroaryl group, wherein there is more than one substituent on the heteroaryl group. In another embodiment, R is an unsubstituted heteroaryl group. In another embodiment, R is an optionally substituted heteroaryl selected from, but not limited to, imidazopyridinyl, pyrazolopyrimidinyl, triazopyridinyl, pyrropyridinyl, pyrazopyridinyl, isoxazolyl, pyridinyl, pyrazolyl, imidazolyl, thiazolyl, isothiazolyl, pyrimidinyl, triazolyl, oxadiazolyl, pyrazinyl, indolyl, isoindolyl, tetrazopyridinyl and pyridonyl.

In an embodiment, R is selected from the following:

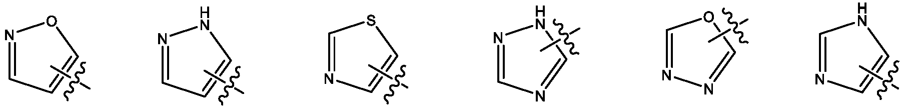

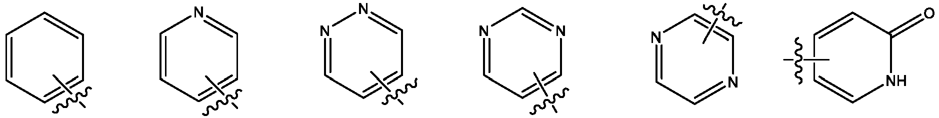

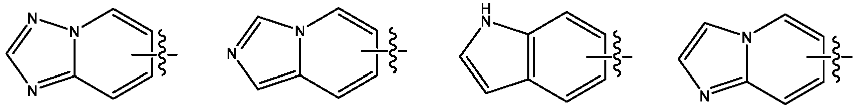

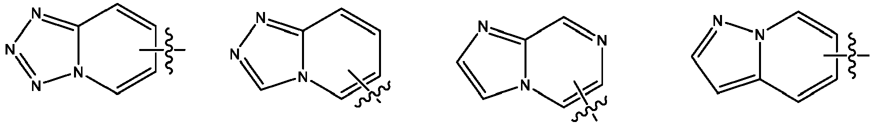

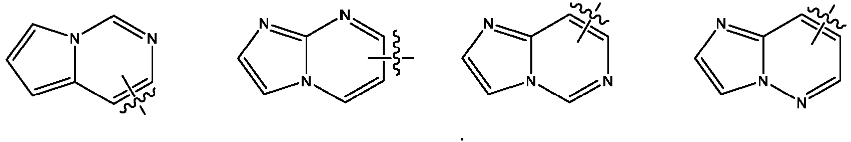

In another embodiment, R is optionally substituted 7-12 membered heteroaryl. In another embodiment, R is selected from:

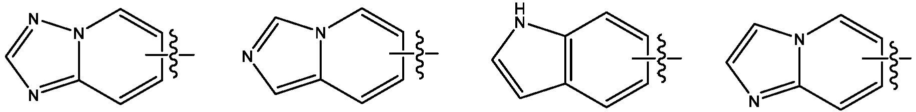

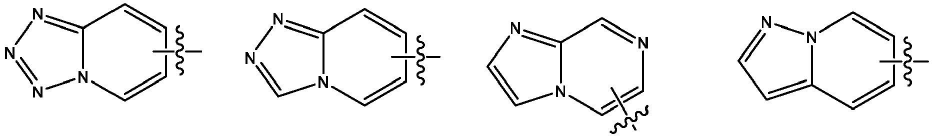

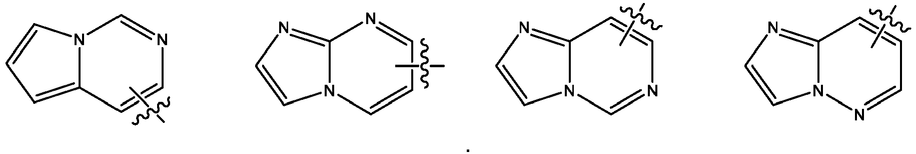

In another embodiment, R is optionally substituted 7-12 membered heteroaryl with 2 or more nitrogen atoms. In another embodiment, R is selected from:

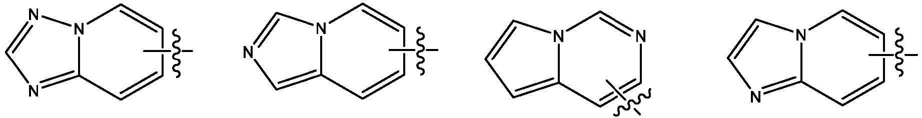

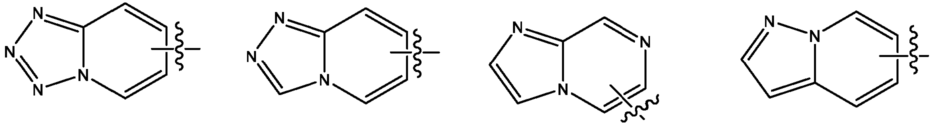

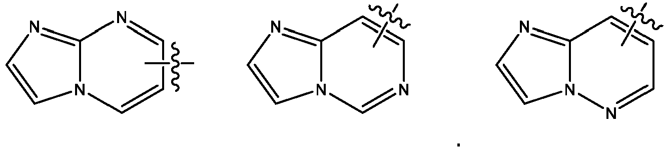

In another embodiment, R is optionally substituted 7-12 membered heteroaryl with 2 nitrogen atoms. In another embodiment, R is selected from:

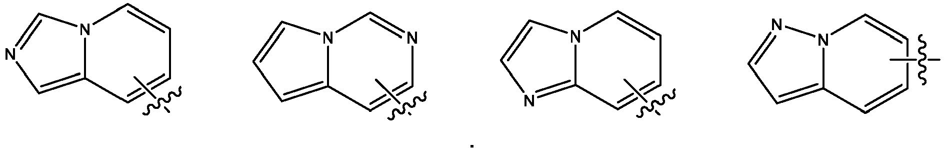

In another embodiment, R is selected from:

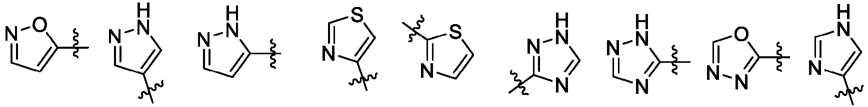

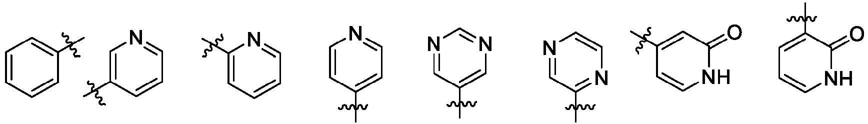

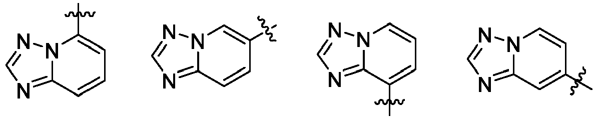

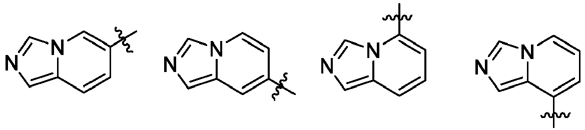

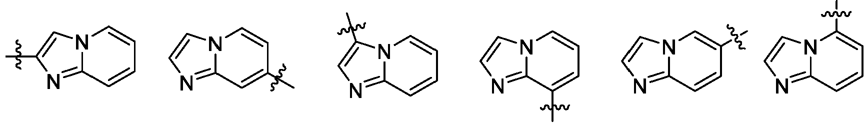

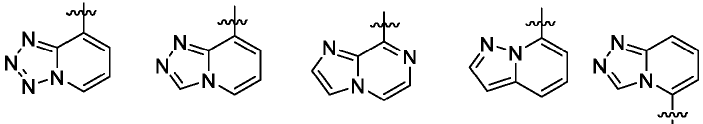

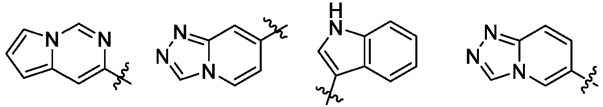

In an embodiment, R¹ is H or optionally substituted C₁-C₄ alkyl. In some embodiments, R¹ is H. In some embodiments, R¹ is optionally substituted C₁-C₄ alkyl. In other embodiments, R¹ is optionally substituted methyl, optionally substituted ethyl, optionally substituted propyl, optionally substituted isopropyl, optionally substituted butyl, optionally substituted sec-butyl, optionally substituted isobutyl or optionally substituted tert-butyl.

In an embodiment, when R¹ is an optionally substituted C₁-C₄ alkyl, the alkyl group has one or more substituent. In an embodiment, R¹ is C₁-C₄ alkyl substituted 1 to 3 times independently selected from halogen (in particular, CI, Br or F), C₁₋₆ alkyl, C₁₋₆ alkoxy, C₂₋₆ alkenyl, C₂₋₆ alkynyl, C₁₋₆ haloalkyl (in particular -CF₃), C₁₋₆ haloalkoxy (such as -OCF₃), -OH, phenyl, benzyl, phenoxy, benzyloxy, benzoyl, -NH₂, -NHC₁₋₄ alkyl, -N(C₁₋₄alkyl)₂, -CN, -NO₂, mercapto, -P=O(OH)(NH₂), -S(O)₂NH₂, -S(O)₂NHC₁₋₄ alkyl, -S(O)₂N(C₁₋₄ alkyl)₂, C₁₋₆ alkylcarbonyl, C₁₋₆ alkoxycarbonyl, CO₂H, -S(O)R‴ (where R‴ is lower alkyl or cycloalkyl) and -S(O)₂R‴ (where R‴ is lower alkyl, cycloalkyl or OH). In some embodiments, R¹ is C₁-C₄ alkyl substituted with C₁-C₄ alkoxy.

In an embodiment, R², R³, R⁴ and R⁵ are independently H or C₁-C₄ alkyl. In some embodiments, R², R³, R⁴ and R⁵ are H. In some embodiments, R², R³, R⁴ and R⁵ are independently C₁-C₄ alkyl. In another embodiment, R², R³, R⁴ and R⁵ are independently H, methyl, ethyl, propyl, isopropyl, butyl, sec-butyl, isobutyl or tertbutyl.

In an embodiment, R² and R³ together form a cycloalkyl ring. In another embodiment, R² and R³ together form a C₃-C₆ cycloalkyl ring. In another embodiment, R² and R³ together form a C₃-C₆ cycloalkyl ring, selected from cyclopropyl, cyclobutyl, cyclopentyl and cyclohexanyl.

In an embodiment, R⁴ and R⁵ together form a cycloalkyl ring. In another embodiment, R⁴ and R⁵ together form a C₃-C₆ cycloalkyl ring. In another embodiment, R⁴ and R⁵ together form a C₃-C₆ cycloalkyl ring, selected from cyclopropyl, cyclobutyl, cyclopentyl and cyclohexanyl.

In an embodiment, Y is selected from 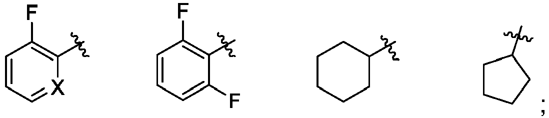 wherein X is CH or N.

In another embodiment, Y is selected from

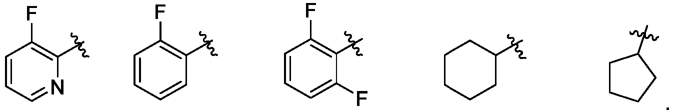

In some embodiments, Y is selected from

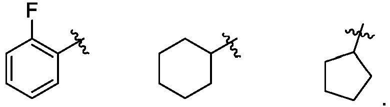

In an embodiment, R is optionally substituted heteroaryl;
R¹ is H or optionally substituted C₁-C₄ alkyl;
R², R³, R⁴ and R⁵ are independently H or C₁-C₄ alkyl; and
Y is selected from

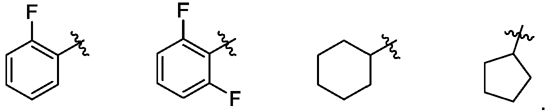

In an embodiment, R is optionally substituted heteroaryl;
R¹ is H or optionally substituted C₁-C₄ alkyl;
R², R³, R⁴ and R⁵ are independently H; and
Y is selected from

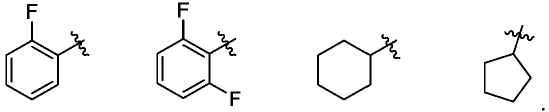

In an embodiment, R is optionally substituted heteroaryl;
R¹ is H or optionally substituted C₁-C₄ alkyl;
R², R³, R⁴ and R⁵ are independently H or methyl; and
Y is selected from

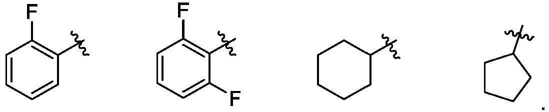

In an embodiment, R is optionally substituted heteroaryl;
R¹, R² and R³ are H;
R⁴ and R⁵ are methyl; and
Y is selected from

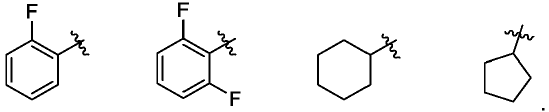

In an embodiment, R is optionally substituted heteroaryl;
R¹, R⁴ and R⁵ are H;
R² and R³ are methyl; and
Y is selected from

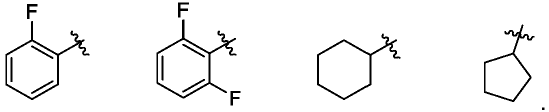

In an embodiment, R is optionally substituted 7-12 membered heteroaryl;
R¹ is H or optionally substituted C₁-C₄ alkyl;
R², R³, R⁴ and R⁵ are independently H or methyl; and
Y is selected from

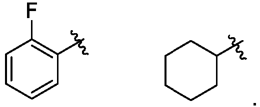

In an embodiment, R is optionally substituted 7-12 membered heteroaryl with 2 or more nitrogen atoms;
R¹ is H or optionally substituted C₁-C₄ alkyl;
R², R³, R⁴ and R⁵ are independently H or methyl; and
Y is selected from

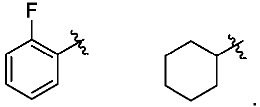

In an embodiment, R is optionally substituted heteroaryl selected from 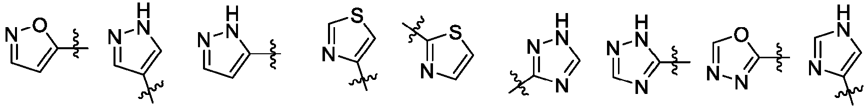 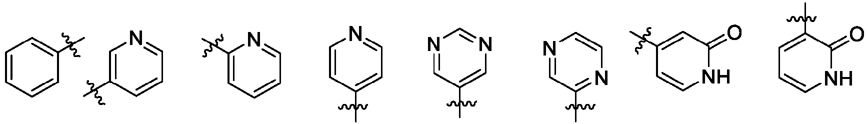 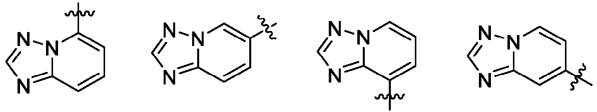 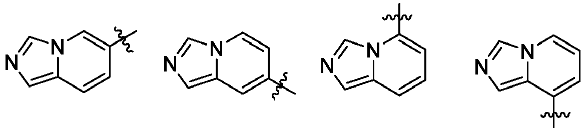 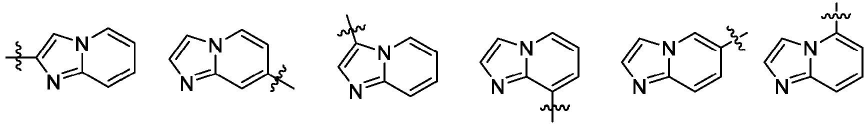 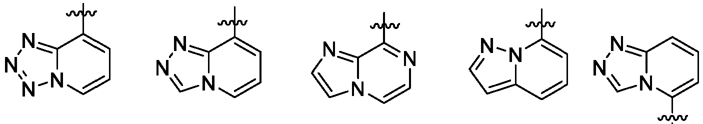 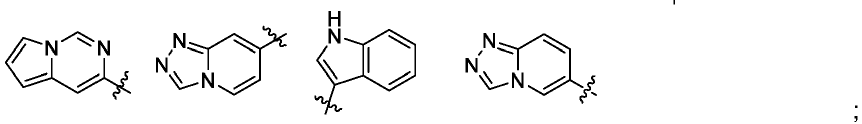
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 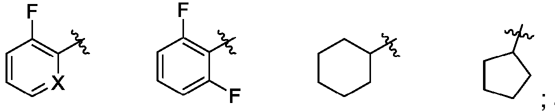 and
X is CH or N.

In an embodiment, R is optionally substituted heteroaryl selected from 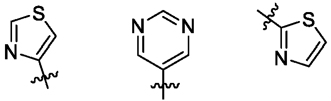 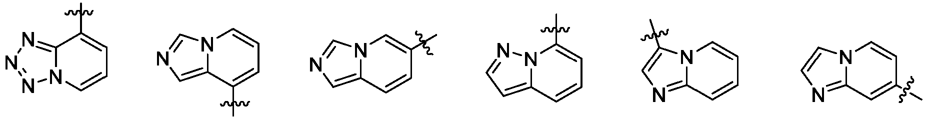 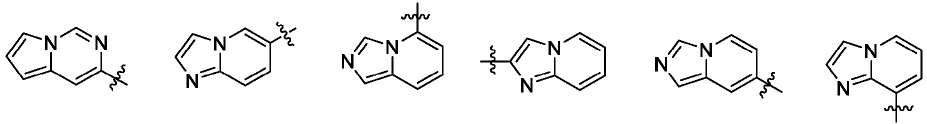 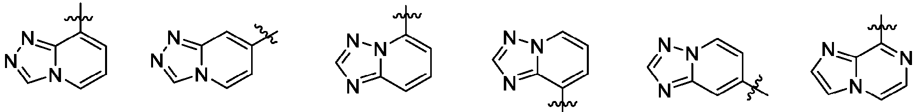 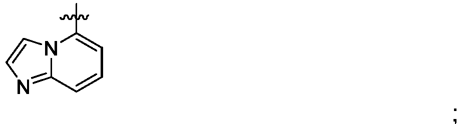
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 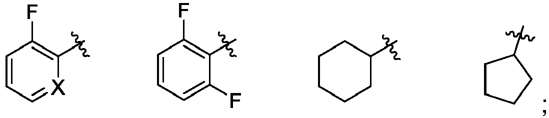 and
X is CH or N.

In an embodiment, R is optionally substituted heteroaryl selected from 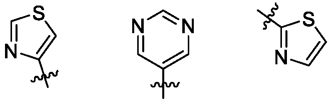 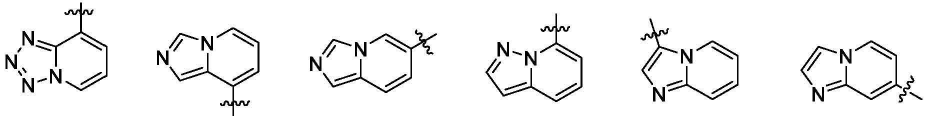 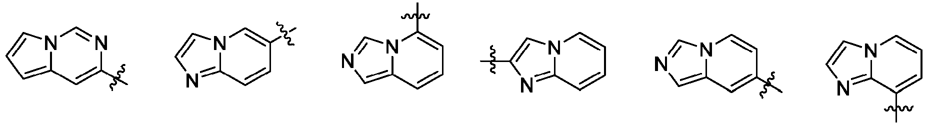 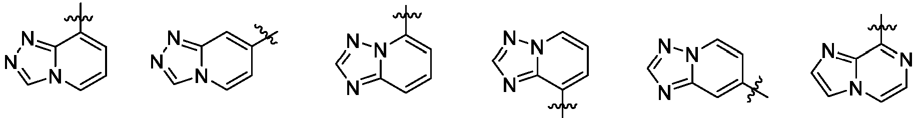 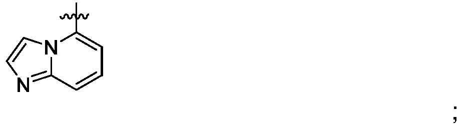
R¹ is H or optionally substituted C₁-C₄ alkyl;
R², R³, R⁴ and R⁵ are independently H or C₁-C₄ alkyl; and
Y is selected from

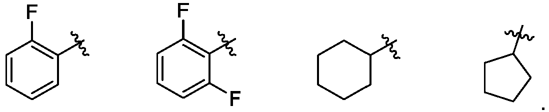

In an embodiment, R is optionally substituted heteroaryl selected from 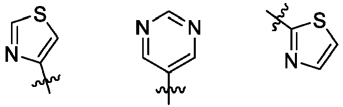 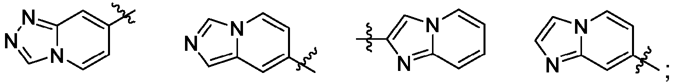
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 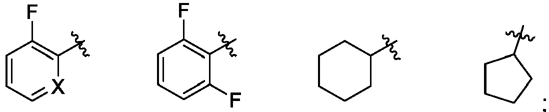 and
X is CH or N.

In an embodiment, R is optionally substituted heteroaryl selected from 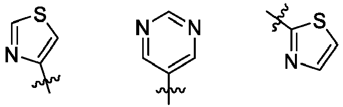 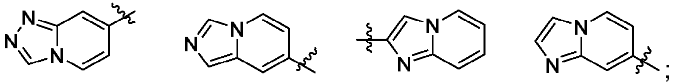
R¹ is H or optionally substituted C₁-C₄ alkyl;
R², R³, R⁴ and R⁵ are independently H or C₁-C₄ alkyl; and
Y is selected from

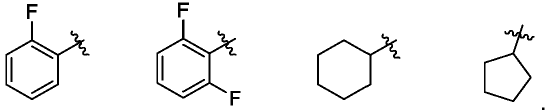

In an embodiment, R is optionally substituted heteroaryl selected from 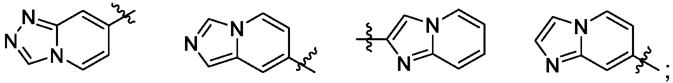
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 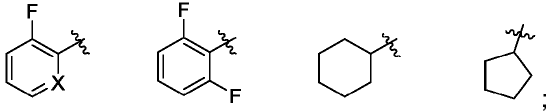 and
X is CH or N.

In an embodiment, R is optionally substituted heteroaryl selected from 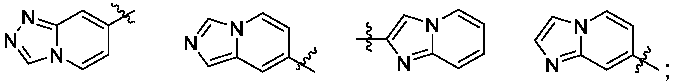
R¹ is H or optionally substituted C₁-C₄ alkyl;
R², R³, R⁴ and R⁵ are independently H or C₁-C₄ alkyl; and
Y is selected from

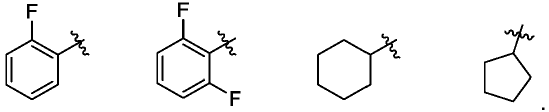

In an embodiment, R is optionally substituted heteroaryl selected from 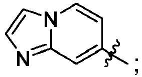
R¹ is H or optionally substituted C₁-C₄ alkyl;
R², R³, R⁴ and R⁵ are independently H or C₁-C₄ alkyl, or R² and R³, or R⁴ and R⁵ together form a cycloalkyl ring;
Y is selected from 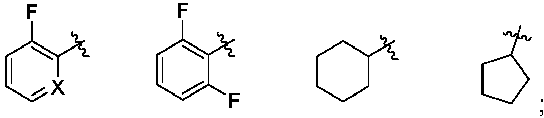 and
X is CH or N.

In an embodiment, when R is optionally substituted heteroaryl selected from 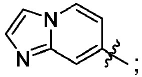
R¹ is H or optionally substituted C₁-C₄ alkyl;
R², R³, R⁴ and R⁵ are independently H or C₁-C₄ alkyl; and
Y is selected from

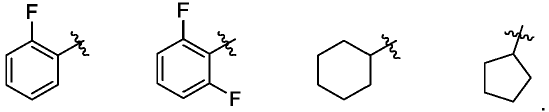

Representative examples of Compounds of Formula (I) include:

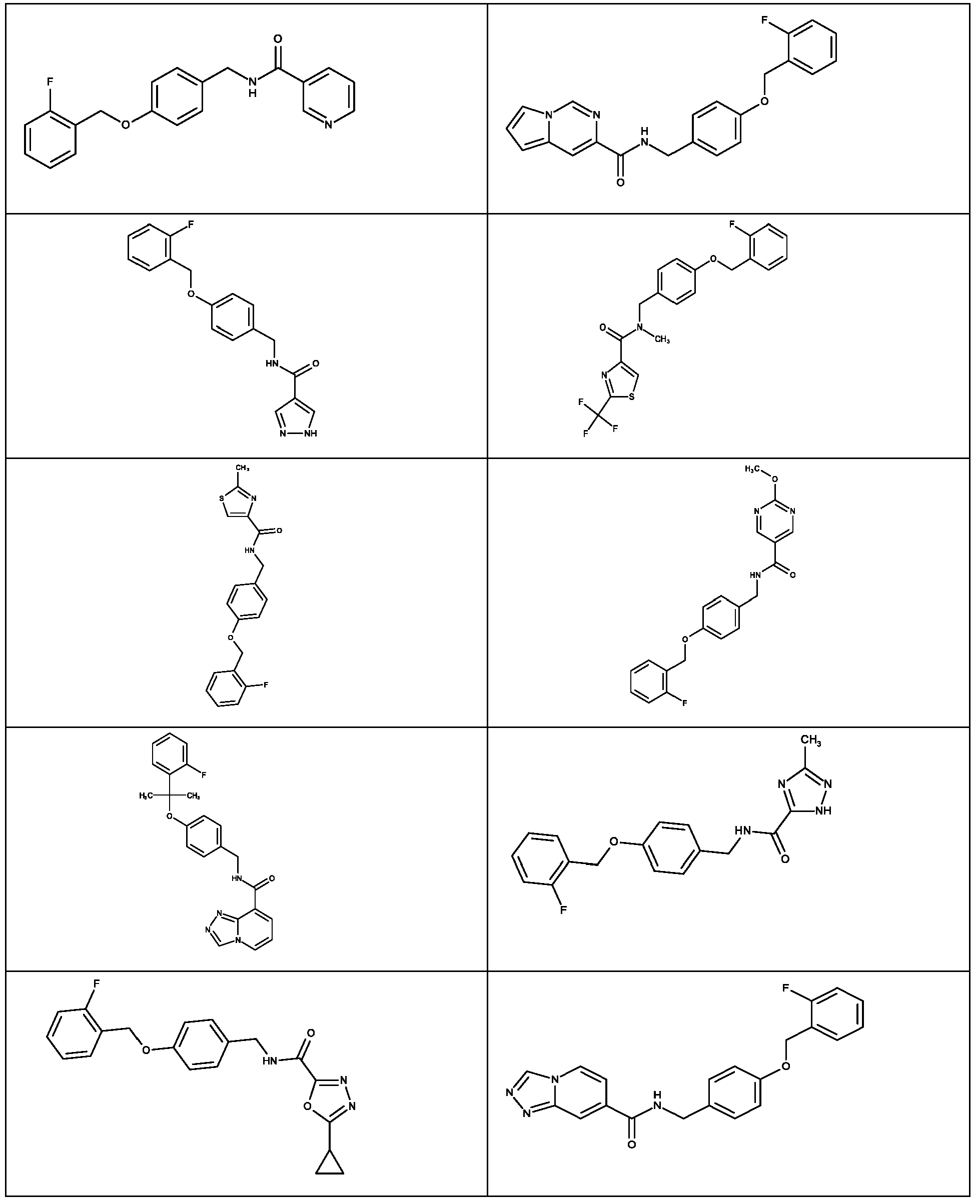

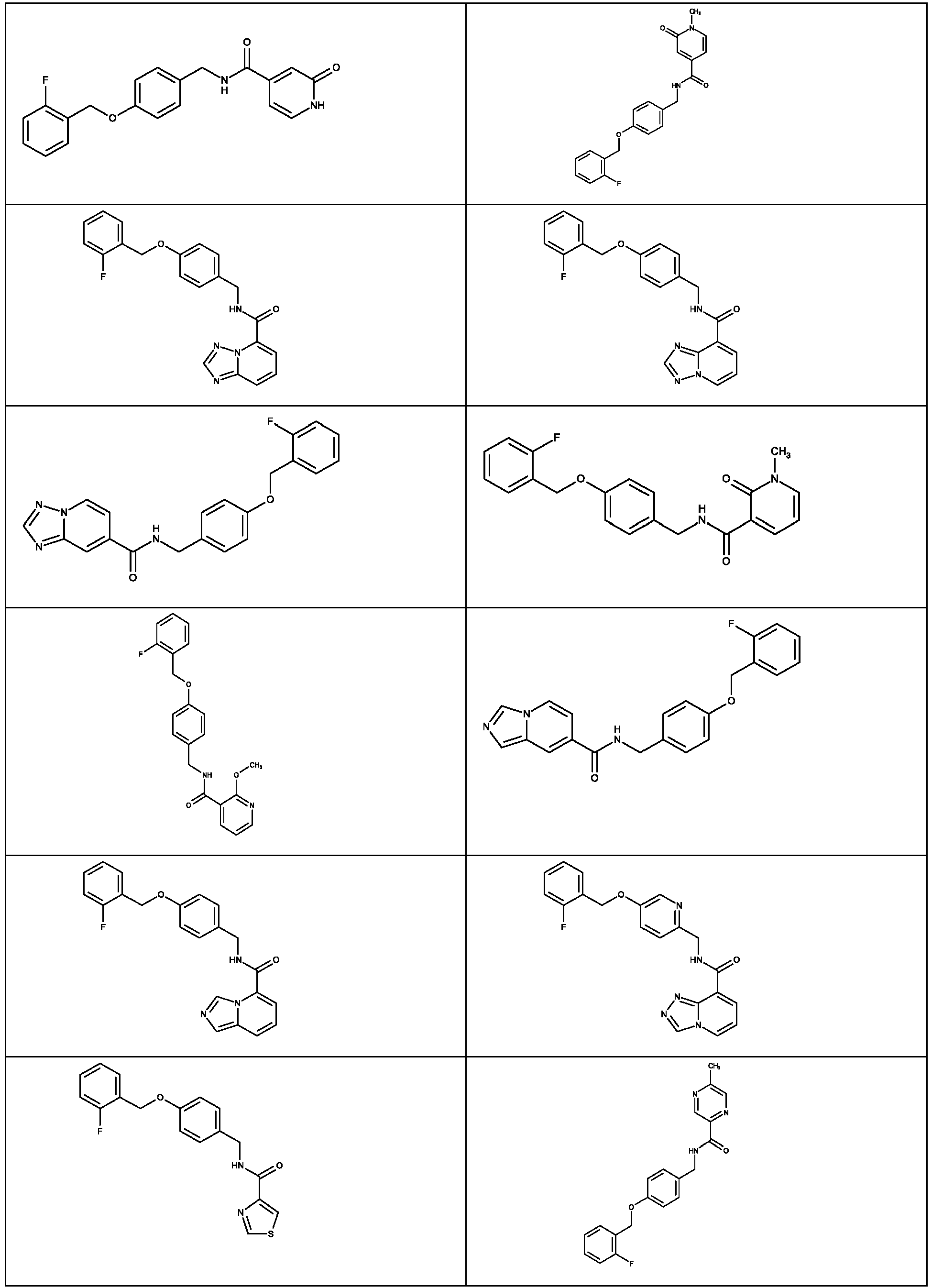

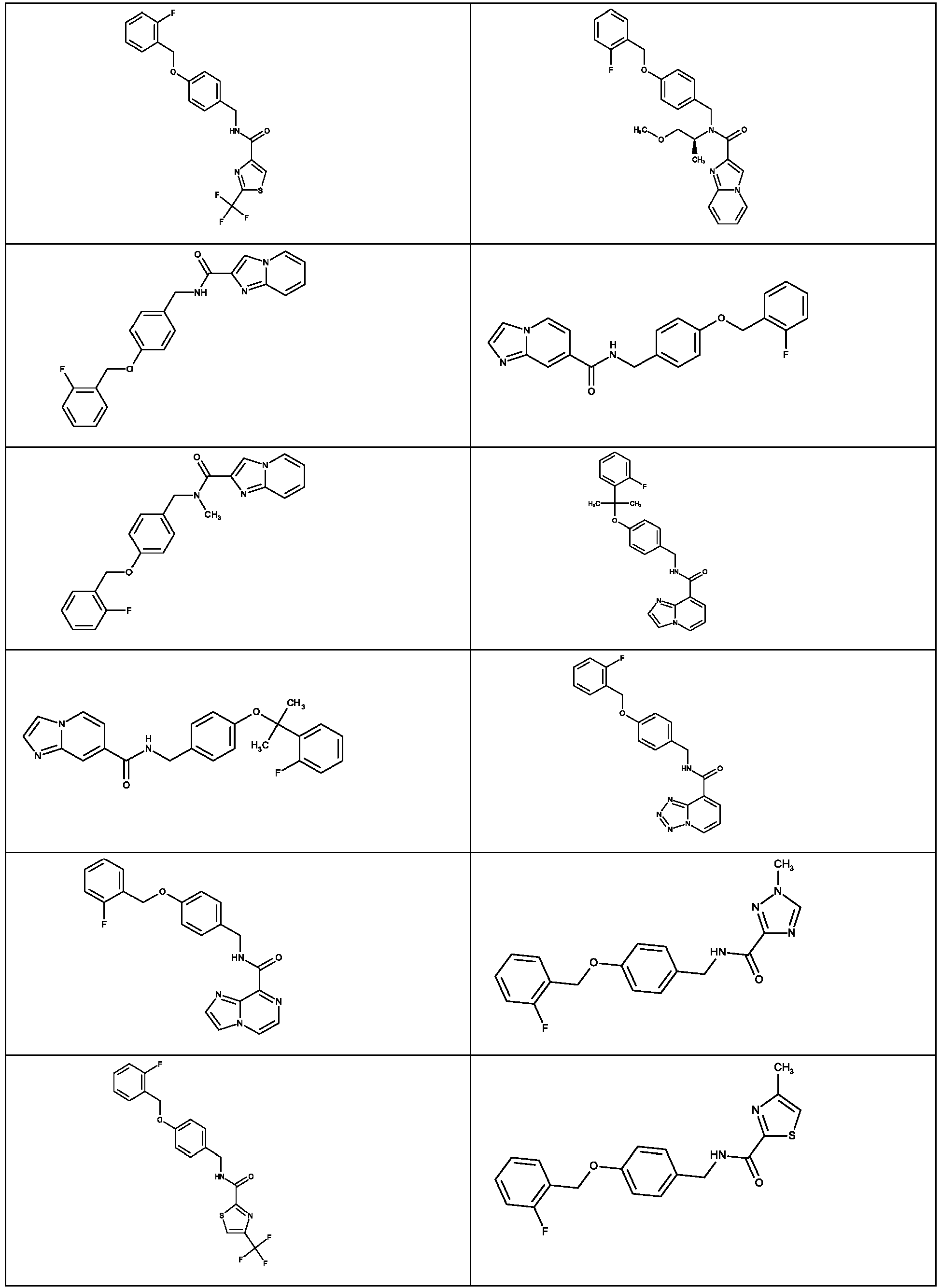

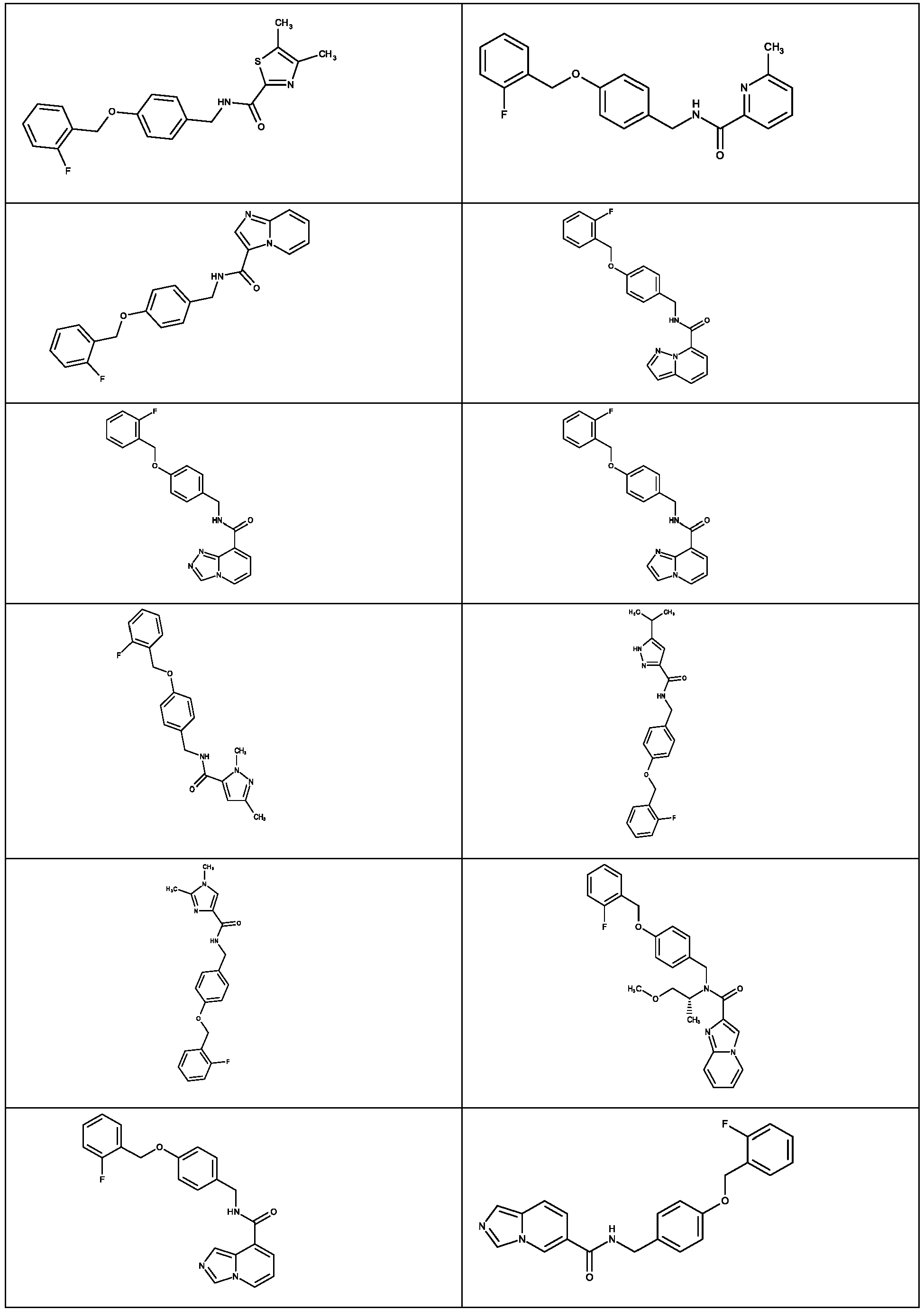

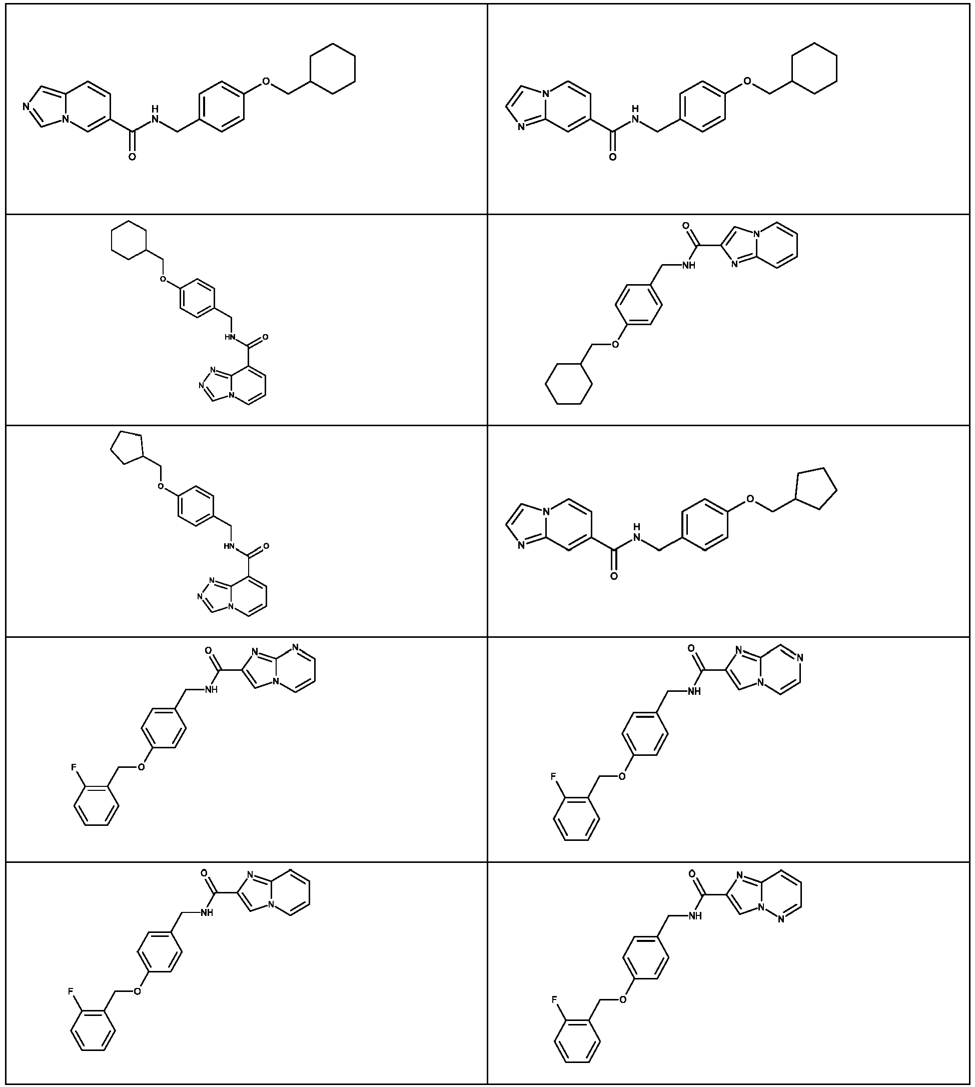

The salts of the compounds of the invention are preferably pharmaceutically acceptable, but it will be appreciated that non-pharmaceutically acceptable salts also fall within the scope of the present invention, since these are useful as intermediates in the preparation of pharmaceutically acceptable salts.

It will be appreciated that the compounds of the invention, and the salts thereof, can be presented in the form of pharmaceutically acceptable solvates and hydrates of the compounds of Formula (I).
(I).

The pharmaceutically acceptable salts include acid addition salts, base addition salts, and the salts of quaternary amines and pyridiniums. The acid addition salts are formed from a compound of the invention and a pharmaceutically acceptable inorganic or organic acid including but not limited to hydrochloric, hydrobromic, sulfuric, phosphoric, methanesulfonic, toluenesulphonic, benzenesulphonic, acetic, propionic, ascorbic, citric, malonic, fumaric, maleic, lactic, salicylic, sulfamic, or tartaric acids. The counter ion of quaternary amines and pyridiniums include chloride, bromide, iodide, sulfate, phosphate, methansulfonate, citrate, acetate, malonate, fumarate, sulfamate, and tartrate. The base addition salts include but are not limited to salts such as sodium, potassium, calcium, lithium, magnesium, ammonium and alkylammonium. Also, basic nitrogen-containing groups may be quaternised with such agents as lower alkyl halides, such as methyl, ethyl, propyl, and butyl chlorides, bromides and iodides; dialkyl sulfates like dimethyl and diethyl sulfate; and others. The salts may be made in a known manner, for example by treating the compound with an appropriate acid or base in the presence of a suitable solvent.

The compounds of the invention may be in crystalline form and/or as solvates (e.g. hydrates) and it is intended that both forms be within the scope of the present invention. The term "solvate" is a complex of variable stoichiometry formed by a solute (in this invention, a compound of the invention) and a solvent. Such solvents should not interfere with the biological activity of the solute. Solvents may be, by way of example, water, ethanol or acetic acid. Methods of solvation are generally known within the art.

Herein disclosed are also "pro-drugs", i.e. compounds
that are converted *in vivo* to the compounds of the invention. Such derivatives would readily occur to those skilled in the art, and include, for example, compounds where a free hydroxy group is converted into an ester derivative or a ring nitrogen atom is converted to an N-oxide. Examples of ester derivatives include alkyl esters, phosphate esters and those formed from amino acids, preferably valine.

The term "pharmaceutically acceptable ester" includes biologically acceptable esters of compound of the invention such as sulphonic, phosphonic and carboxylic acid derivatives.

Thus, in another aspect of the invention, there is provided a prodrug or pharmaceutically acceptable ester of a compound of the invention or of salt thereof.

It will be appreciated that the compounds of the invention may have at least one asymmetric centre, and therefore are capable of existing in more than one stereoisomeric form. The invention extends to each of these forms individually and to mixtures thereof, including racemates. The isomers may be separated conventionally by chromatographic methods or using a resolving agent. Alternatively the individual isomers may be prepared by asymmetric synthesis using chiral intermediates. Where the compound has at least one carbon-carbon double bond, it may occur in Z- and E- forms with all isomeric forms of the compounds being included in the present invention.

The invention also includes where possible a salt or pharmaceutically acceptable derivative such as a pharmaceutically acceptable ester, solvate and/or prodrug of the above mentioned embodiments of the invention.

In another aspect of the invention, there is provided a pharmaceutical composition that comprises a therapeutically effective amount of one or more of the aforementioned compounds or pharmaceutically acceptable salts thereof, including pharmaceutically acceptable derivatives thereof, and optionally a pharmaceutically acceptable carrier or diluent.

In another aspect, the present invention provides pharmaceutical compositions for use as a sodium ion channel modulators, more particularly as pain relief agents, the composition comprising an effective amount of a compound of Formula (I), or a pharmaceutically acceptable salt thereof, including a pharmaceutically acceptable derivative thereof, and optionally a pharmaceutically acceptable carrier or diluent.

The term "composition" is intended to include the formulation of an active ingredient with encapsulating material as carrier, to give a capsule in which the active ingredient (with or without other carrier) is surrounded by carriers.

The pharmaceutical compositions or formulations include those suitable for oral, rectal, nasal, topical (including buccal and sub-lingual), vaginal or parenteral (including intramuscular, sub-cutaneous and intravenous) administration or in a form suitable for administration by inhalation or insufflation.

The compounds of the invention, together with a conventional adjuvant, carrier, or diluent, may thus be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use.

Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Formulations containing ten (10) milligrams of active ingredient or, more broadly, 0.1 to one hundred (100) milligrams, per tablet, are accordingly suitable representative unit dosage forms.

The compounds of the present invention can be administered in a wide variety of oral and parenteral dosage forms. It will be obvious to those skilled in the art that the following dosage forms may comprise, as the active component, either a compound of the invention or a pharmaceutically acceptable salt of a compound of the invention.

For preparing pharmaceutical compositions from the compounds of the present invention, pharmaceutically acceptable carriers can be either solid or liquid. Solid form preparations include powders, tablets, pills, capsules, cachets, suppositories, and dispensable granules. A solid carrier can be one or more substances which may also act as diluents, flavouring agents, solubilisers, lubricants, suspending agents, binders, preservatives, tablet disintegrating agents, or an encapsulating material.

In powders, the carrier is a finely divided solid that is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized moulds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution.

Sterile liquid form compositions include sterile solutions, suspensions, emulsions, syrups and elixirs. The active ingredient can be dissolved or suspended in a pharmaceutically acceptable carrier, such as sterile water, sterile organic solvent or a mixture of both.

The compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilisation from solution, for constitution with a suitable vehicle, eg. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations that are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilising agents, and the like.

For topical administration to the epidermis the compounds according to the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Formulations suitable for topical administration in the mouth include lozenges comprising active agent in a flavoured base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multidose form. In the latter case of a dropper or pipette, this may be achieved by the patient administering an appropriate, predetermined volume of the solution or suspension. In the case of a spray, this may be achieved for example by means of a metering atomising spray pump. To improve nasal delivery and retention the compounds according to the invention may be encapsulated with cyclodextrins, or formulated with other agents expected to enhance delivery and retention in the nasal mucosa.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In formulations intended for administration to the respiratory tract, including intranasal formulations, the compound will generally have a small particle size for example of the order of 5 to 10 microns or less. Such a particle size may be obtained by means known in the art, for example by micronisation.

When desired, formulations adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The invention also includes the compounds in the absence of carrier where the compounds are in unit dosage form.

The amount of the compound of the invention to be administered may be in the range from about 10 mg to 2000 mg per day, depending on the activity of the compound and the disease to be treated.

Liquids or powders for intranasal administration, tablets or capsules for oral administration and liquids for intravenous administration are the preferred compositions.

The pharmaceutical preparations of the compounds according to the present invention may be co-administered with one or more other active agents in combination therapy. For example the pharmaceutical preparation of the active compound may be co-administered (for example, separately, concurrently or sequentially), with one or more other agents used to treat cognitive impairment or mood disorders such as acetylcholine esterase inhibitors, antipsychotics, and antidepressants.

### GENERAL SYNTHETIC SCHEMES AND DESCRIPTION

### GENERAL PROCEDURES

### EXAMPLES

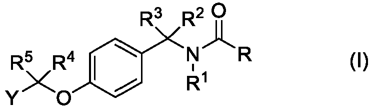

### Acronyms

For convenience, many chemical moieties are represented using well known abbreviations, including but not limited to, methyl (Me), ethyl (Et), n-propyl (nPr), *iso*propyl (iPr), *n*-butyl (nBu), *tert*-butyl (tBu), *n*-hexyl (nHex), cyclohexyl (cHex), phenyl (Ph), methoxy (MeO), ethoxy (EtO), trimethylsilyl (TMS), *tert*-butyloxycarbonyl (Boc), and acetyl (Ac).

For convenience, many chemical compounds are represented using well known abbreviations, including but not limited to, methanol (MeOH), ethanol (EtOH), ether or diethyl ether (Et₂O), ethyl acetate (EtOAc), triethylamine (EtsN), dichloromethane (methylene chloride, DCM), trifluoroacetic acid (TFA), trifluoroethanol (TFE), dimethylformamide (DMF), sodium sulphate (Na₂SO₄), tetrahydrofuran (THF), *meta-*chloroperbenzoic acid (*m*CPBA), hexamethyldisilazane sodium salt (NaHMDS), *O-*(7-azabenzotriazol-1-yl)-N,N,N',N'-tetramethyluronium hexafluorophosphate (HATU), dimethylsulfoxide (DMSO), magnesium sulphate (MgSO₄), sodium hydrogen carbonate (NaHCOs), *tert*-butanol (*t*-BuOH), 1-ethyl-3-(3-dimethylaminopropyl) carbodiimide hydrochloride salt (EDCI.HCl), tetra-*n*-butylammonium fluoride (TBAF), *N,N*-diisopropylethylamine (DIPEA), 1-hydroxybenzotriazole (HOBt), *trans-*dichlorobis(triphenylphosphine)palladium(II) (PdCl₂(PPh₃)₂), tetrakis(triphenylphosphine)palladium(0) (Pd(PPh₃)₄) tris(dibenzylideneacetone) dipalladium(0) (Pd₂(dba)₃), tri-t-butyl phosphonium tetrafluoroborate (*t*-Bu₃PH.BF₄), 4,5-bis(diphenylphosphino)-9,9-dimethylxanthene (Xantphos), triphenylphosphine (PPhs), diisopropyl azodicarboxylate (DIAD), pyridinium chlorochromate (PCC), borane dimethylsulfide (BMS), titanium isopropoxide (TiOiPr₄), sodium triacetoxyborohydride (NaBH(OAc)₃), sodium cyanoborohydride (NaBH₃(CN)), ammonium chloride (NH₄Cl), chloroform (CHCl₃), manganese dioxide (MnO₂), potassium carbonate (K₂CO₃) and 1,2-dichloroethane (DCE).

### General Experimental Details

Unless otherwise stated the following generalisations apply.

NMR, HPLC, MS and Mp data provided in the examples described below are registered on:
NMR: Agilent DD2 (500 MHz), Aglient DD2 (600 MHz) or Varian DD2 (300 MHz) using residual signal of deuterated solvent as internal reference.

LCMS: Agilent Technologies LC/MS (1260 Infinity, 6120 Quadrupole LC/MS), column Zorbax SB-C8, 4.6 x 150mm, 5µ, with mobile phase 80% ACN, 15% H₂O, 5% buffer (3:1 MeOH/H₂O, 315 mg HCO₂NH₄, 1 mL AcOH) and MS detection (ESI method).

### Mp: SRS OptiMelt - Automated Melting Point System

Analytical thin-layer chromatography (TLC) was performed on Merck silica gel 60F254 aluminium-backed plates which were visualised using fluorescence quenching under UV light or using an acidic anisaldehyde or a basic potassium permanganate dip. Flash chromatography was performed using either a Teledyne Isco CombiFlash Rf purification system using standard RediSep^{®} cartridges. Microwave irradiation was achieved using a CEM Explorer 48 Microwave Reactor. All reactions carried out using microwave irradiation were stirred.

Where necessary, anhydrous solvents were prepared using a Glascontour purification system or purchased from Sigma-Aldrich.

### A - General procedure for (4-(cycloalkylmethoxy)phenyl)methanamine synthesis

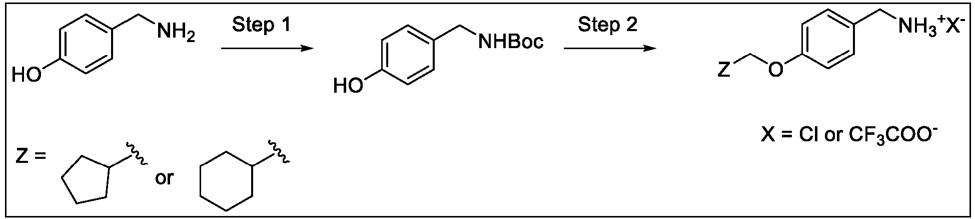

### Step 1:

To a solution of 4-hydroxybenzylamine in dry dichloromethane (10mL) under N₂ was added triethylamine (2eq) followed by di-tert-butyl carbonate (2eq). The reaction was allowed to stir at rt for 3h before being quenched with saturated NH₄Cl solution and then extracted with EtOAc (2x). The extracts were combined and dried over MgSO₄, filtered and concentrated in vacuo. The crude material was taken up in MeOH and treated with K₂CO₃ (excess) at rt overnight. The reaction mixture was concentrated in vacuo and 10% citric acid solution was added. The mixture was extracted with EtOAc (2x). The extracts were combined and dried over MgSO₄, filtered and concentrated in vacuo. The crude material was purified by flash column chromatography on silica gel using an ethyl acetate/dichloromethane gradient as eluent.

### Step 2:

The (bromoalkyl)cycloalkane (1.1eq), caesium carbonate (1.2eq) and the phenol from step 1 were combined in a flask under N₂ and taken up in dry DMF or DMA (10mL). The reaction was stirred at elevated temperature (for example 70°C) overnight. The reaction was quenched with saturated NH₄Cl solution and extracted with EtOAc (2x). The extracts were combined, washed with water (x3) and brine and dried over MgSO₄, filtered and concentrated in vacuo. The crude material was purified by flash column chromatography on silica gel. The resulting product was taken up in either 4M hydrochloric acid in dioxane or trifluoroacetic acid (excess) and dichloromethane (6mL) and the mixture stirred at rt for 4 h. The reaction mixture was concentrated in vacuo and to the residue was added diethyl ether. The resulting solid was collected by vacuum filtration and dried in vacuo to give the benzylamine salt.

### B - Synthesis of (5-((2-fluorobenzyl)oxy)pyridin-2-yl)methanamine hydrochloride:

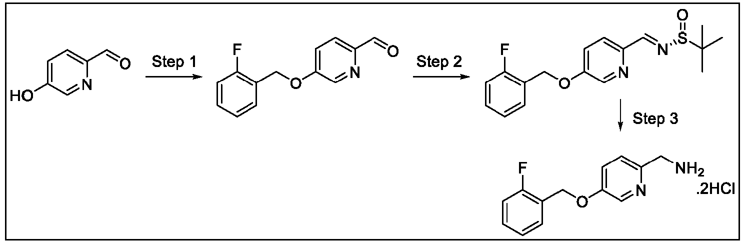

### Step 1:

Hydroxypyridine-2-carbaldehyde (499 mg, 4.05 mmol), 2-fluorobenzyl bromide (500uL) and potassium carbonate (691 mg, 5.00mmol) were combined in a flask under N₂ and taken up in dry DMF (10mL). The reaction was stirred at 60°C overnight. The reaction was quenched with water and extracted with EtOAc (2x). The extracts were combined, washed with water (x3) and brine and dried over MgSO₄, filtered and concentrated in vacuo. The crude material was purified by flash column chromatography on silica gel using a diethyl ether dichloromethane gradient as eluent to give the desired product (665mg, 71%).

### Step 2:

(R)-(+)-2-methyl-2-propanesulfinamide (380mg, 3.13mmol) and the product from step 1 were combined in a flask under N₂ and taken up in dry THF (10mL). Titanium ethoxide (1.2mL, 5.72mmol) was added and the reaction was stirred at rt over the weekend. A further portion of the sulfinamide (103mg, 0.846mmol) was added and the reaction was stirred at 60°C overnight. The mixture was diluted with ethyl acetate and quenched with minimal saturated sodium bicarbonate solution before filtration through a short pad of celite, which was rinsed with ethyl acetate. The filtrate was evaporated and the crude material was purified by flash chromatography on silica gel using a ethyl acetate/dichloromethane gradient as eluent to give the desired product (700mg, 73%).

### Step 3:

The product from step 2 was taken up in ethanol (5mL), cooled to 0°C and treated with sodium borohydride (87.9mg, 2.32mmol). The reaction was allowed to achieve ambient temperature over 5 hours. The reaction was concentrated in vacuo and the crude material partitioned between saturated ammonium chloride and ethyl acetate. The layers were separated and the aqueous further extracted with EtOAc. The extracts were combined, dried over MgSO₄, filtered and concentrated in vacuo. The crude material was purified by flash chromatography on silica gel using an ethyl acetate/dichloromethane gradient followed by a methanol/ethyl acetate gradient as eluent. The material thus obtained was taken up in 4M HCl in dioxane (8mL). MeOH (8mL) was added and the reaction was stirred at room temperature for 3h. The reaction was concentrated in vacuo and the crude material triturated with dry diethyl ether and dried in vacuo to give the product as a hydrochloride salt (470mg, 75% over two steps).

### C - Synthesis of [4-[(2-fluorophenyl)methoxy]phenyl]methanamine:

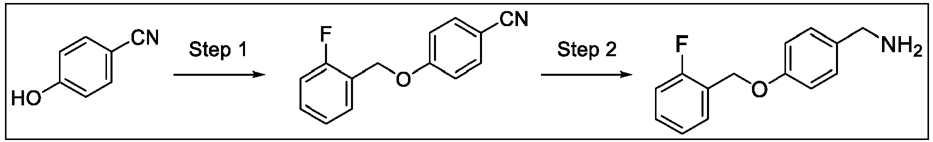

### Step 1:

Sodium carbonate (22.6 g, 213 mmol) was added to a solution of 2-fluorobenzylbromide (6.4 mL, 53.4 mmol) and 4-cyanophenol (7.0 g, 58.8 mmol) in acetone (280mL). The mixture was stirred at 60°C for 18 hours. The mixture was then concentrated, water was added and the mixture was extracted with EtOAc (2x). The extracts were combined and dried over MgSO₄, filtered and concentrated in vacuo. The crude material was purified by flash column chromatography on silica gel using an ethyl acetate cyclohexane gradient as eluent to afford the expected compound as a white solid (10.0 g, 83% yield).

### Step 2:

Lithium aluminium hydride (5.0 g, 132mmol) was suspended in THF (400mL) and was cooled to 0°C. A solution of 4-[(2-fluorophenyl)methoxy]benzonitrile (10.0 g, 44.0 mmol) in THF (100mL) was added dropwise at 0°C. The mixture was stirred and allowed to warm to room temperature over 18h. The reaction mixture was then cooled to 0°C and water (5mL) was added dropwise. After stirring at 0°C for 15 min, 2M sodium hydroxide solution (5mL) was added followed by water (15mL). The mixture was stirred at 0°C for 1h before filtration through a short pad of celite, which was rinsed with dichloromethane and ethyl acetate. The filtrate was concentrated to afford the expected compound as white oil (9.6 g, 95% yield).

### D - Preparation of [4-[1-(2-fluorophenyl)-1-methyl-ethoxy]phenyl]methanamine

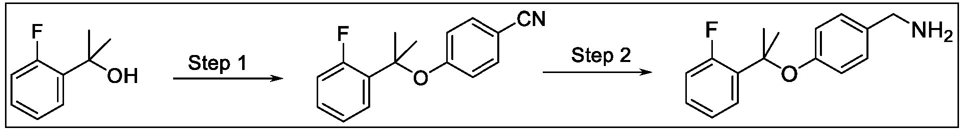

### Step 1: Synthesis of 4-[1-(2-fluorophenyl)-1-methyl-ethoxy]benzonitrile

Sodium hydride (60% in oil, 572 mg, 14.3 mmol) was added at 0°C to a solution of 2-(2-Fluorophenyl)propan-2-ol (2.0 g, 13 mmol) in DMF (15mL). The mixture was stirred at 0°C for 15 min. 4-fluorobenzonitrile (1.6 g, 13 mmol, 1 eq) was then added and the mixture warmed to room temperature for 30 min then stirred at 50°C for 20h. After cooling, water was added followed by saturated solution of ammonium chloride before extraction with ethyl acetate. The organic layers were dried over magnesium sulfate, filtered and concentrated under vacuo. The residue was purified by flash chromatography over silica gel using an ethyl acetate/cyclohexane gradient as eluent afford the expected compound as white solid (1.89 g, 57% yield)

### Step 2: Synthesis of [4-[1-(2-fluorophenyl)-1-methyl-ethoxy]phenyl]methanamine

A lithium aluminium hydride solution (2M in THF, 11.1 mL, 22.2 mmol) was added dropwise at 0°C to a solution of 4-[1-(2-fluorophenyl)-1-methyl-ethoxy]benzonitrile (1.89 g, 7.40 mmol) in THF (15mL). The mixture was stirred from 0°C to room temperature for 3 days. The reaction mixture was then cooled down to 0°C and water (0.84 mL) was added carefully. After stirring 15 min at 0°C, 2M solution sodium hydroxide (0.84 mL) was added followed by water (2.5mL). The mixture was stirred at 0°C during 15 min then filtered through a short pad of celite and rinsed with methylene chloride and ethyl acetate. The filtrate was evaporated to afford the expected compound as light-yellow oil (1.74 g, 91% yield).

### E - General Procedure for Amide Couplings:

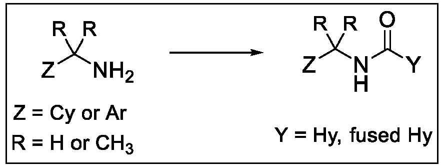

Amide couplings were performed using standard methods. In general, the benzylamine or the salt thereof and the carboxylic acid (1.05eq) were combined in a flask under N₂ and taken up in a solvent such as THF, DMF, or 1,2-dichloroethane (10mL) or a combination of solvents (for example THF and DMF). A base (1-2.5eq), such as diisopropylethylamine, was added followed by an amide coupling reagent (1-2eq) (for example hexafluorophosphate azabenzotriazole tetramethyl uronium, 50% propylphosphonic anhydride in ethyl acetate or [1-ethyl-3-(3-dimethylaminopropyl) carbodiimide hydrochloride] in combination with hydroxybenzotriazole). The mixture was stirred at room temperature or at elevated temperature (for example 50°C) overnight or until the reaction was complete. In some cases, microwave irradiation was required to affect the desired amide formation (for example irradiation at 140°C for 1h). The reaction was quenched with either saturated NH₄Cl or saturated NaHCOs solution and then extracted with solvent (for example ethyl acetate), dried over magnesium sulfate, filtered and evaporated. The residue was purified by flash chromatography on silica gel.

### F - General Procedure for Amide Couplings:

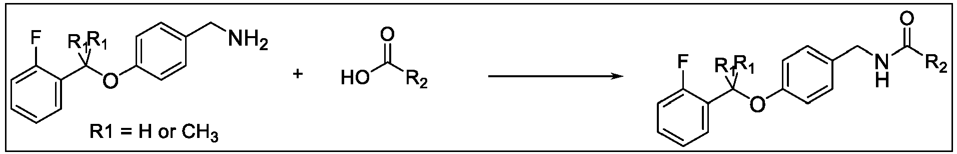

At 0°C, to a suspension or a solution of the carboxylic acid (1 eq) in a mixture of THF/DMF was successively added the benzylamine (1 - 1.2 eq), diisopropylethylamine (3 eq), EDCI.HCl (1.5 eq) and HOBt.H₂O (1.5 eq). The mixture was stirred at room temperature from overnight to several days. Then, a saturated solution of ammonium chloride was added before extraction with ethyl acetate. The organic layers were dried over magnesium sulfate, filtered and evaporated under vacuo. In some cases, the crude compound was collected directly by filtration from the reaction mixture and washed with water. The desired amides were isolated in pure form after flash chromatography over silica gel (followed by sephadex or/and preparative HPLC if necessary) with yields from 32 to 90%.

**Table 1.**

| The below Compounds of Numbers 1, 3 to 6, 8, 9, 11, 12, 16, 17, 21 to 23, 32 to 36 and 41 to 4 are according to the invention if used for treating or preventing pain disorders. | | | | | | |
|---|---|---|---|---|---|---|
| Number | Structure | Mol Weight | Mass (M+H) | Mass (M-H) | Synthesis - Benzylamine | Synthesis - Amide coupling |
| 1 | 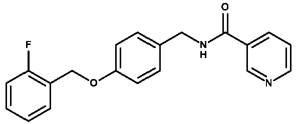 | 336.37 | 337.2 | | C | F |
| 2 | 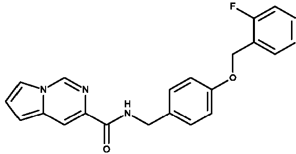 | 375.40 | 376.1 | | C | F |
| 3 | 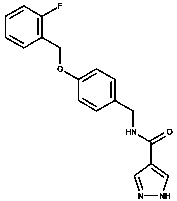 | 325.34 | 326.10 | | C | E |
| 4 | 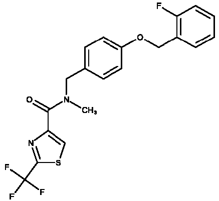 | 424.41 | 425.1 | | C | F |
| 5 | 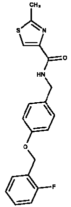 | 356.42 | 357.10 | | C | E |
| 6 | 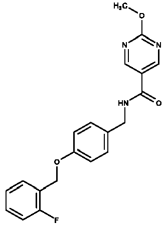 | 367.38 | 368.2 | | C | F |
| 7 | 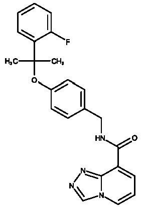 | 404.45 | 405.33 | | D | F |
| 8 | 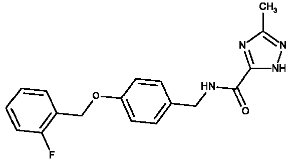 | 340.36 | 341.20 | | C | E |
| 9 | 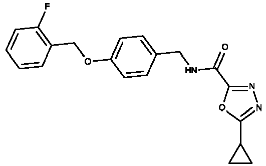 | 367.38 | 368.10 | | C | E |
| 10 | 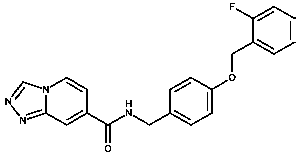 | 376.39 | 377.10 | | C | E |
| 11 | 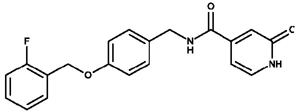 | 352.37 | 353.10 | | C | E |
| 12 | 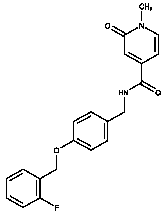 | 366.39 | 367.10 | | C | E |
| 13 | 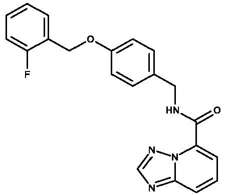 | 376.39 | 377.10 | | C | E |
| 14 | 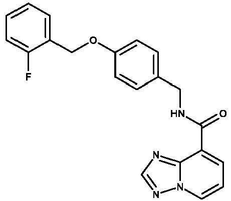 | 376.39 | 377.10 | | C | E |
| 15 | 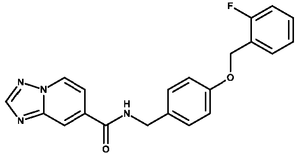 | 376.39 | 377.10 | | C | E |
| 16 | 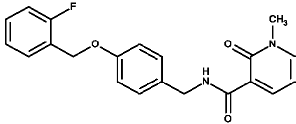 | 366.39 | 367.10 | | C | E |
| 17 | 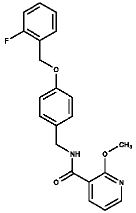 | 366.39 | 367.10 | | C | E |
| 18 | 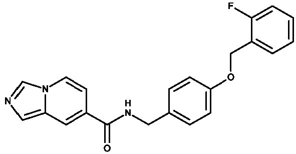 | 375.40 | 376.10 | | C | E |
| 19 | 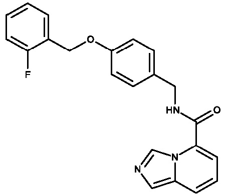 | 375.40 | 376.20 | | C | E |
| 20 | 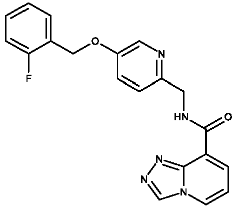 | 377.38 | 378.10 | | B | E |
| 21 | 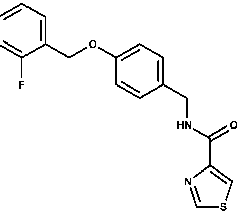 | 342.39 | 343.10 | | C | E |
| 22 | 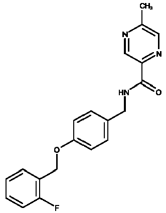 | 351.38 | | 350.00 | C | E |
| 23 | 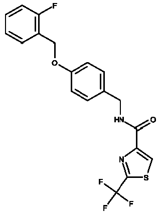 | 410.39 | 411.1 | | C | F |
| 24 | 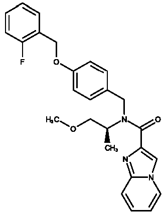 | 447.51 | 448.20 | | C | F |
| 25 | 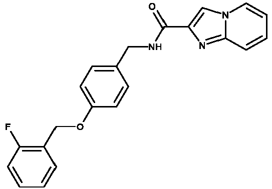 | 375.40 | 376.2 | | C | F |
| 26 | 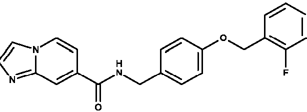 | 375.40 | 376.10 | | C | E |
| 27 | 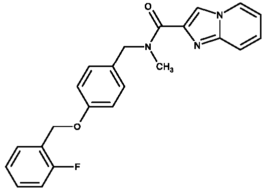 | 389.43 | 390.1 | | C | F |
| 28 | 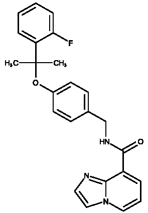 | 403.46 | 404.25 | | D | F |
| 29 | 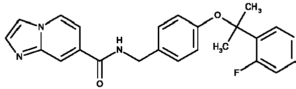 | 403.46 | 404.33 | | D | F |
| 30 | 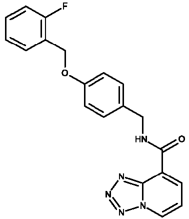 | 377.38 | | 376.00 | C | E |
| 31 | 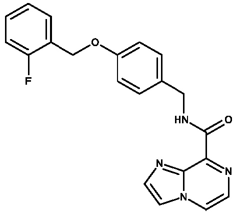 | 376.39 | 377.10 | | C | E |
| 32 | 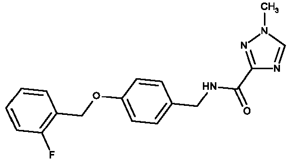 | 340.36 | 341.20 | | C | E |
| 33 | 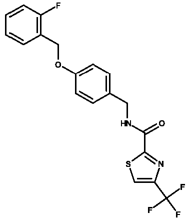 | 410.39 | 411.10 | | C | E |
| 34 | 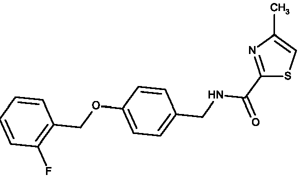 | 356.42 | 357.10 | | C | E |
| 35 | 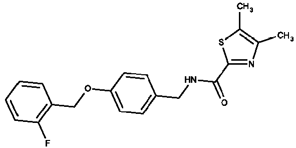 | 370.44 | 371.10 | | C | E |
| 36 | 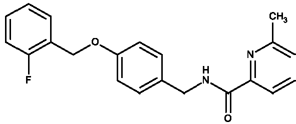 | 350.39 | 351.1 | | C | F |
| 37 | 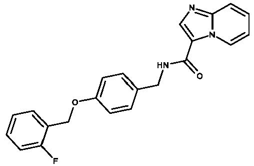 | 375.40 | 376.2 | | C | F |
| 38 | 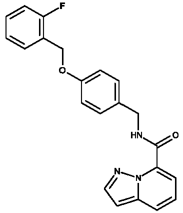 | 375.40 | 376.10 | | C | E |
| 39 | 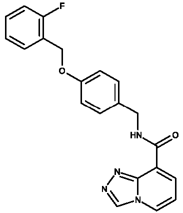 | 376.39 | 377.10 | | C | E |
| 40 | 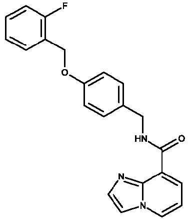 | 375.40 | 376.10 | | C | E |
| 41 | 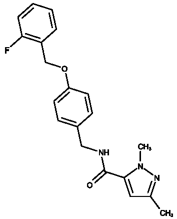 | 353.40 | 354.10 | | C | E |
| 42 | 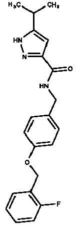 | 367.42 | 91.80 | | C | E |
| 43 | 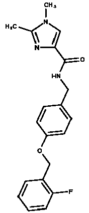 | 353.40 | 354.10 | | C | E |
| 44 | 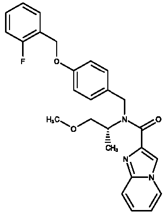 | 447.51 | 448.20 | | C | E |
| 45 | 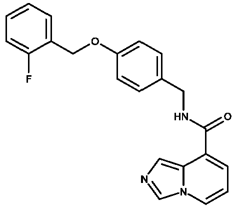 | 375.40 | 376.10 | | C | E |
| 46 | 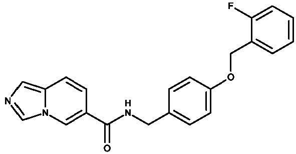 | 375.40 | 376.10 | | C | E |
| 47 | 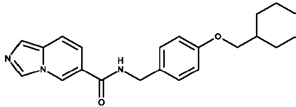 | 363.46 | 364.20 | 362.10 | A | F |
| 48 | 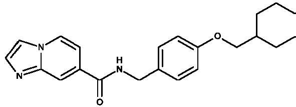 | 363.46 | 364.20 | | A | E |
| 49 | 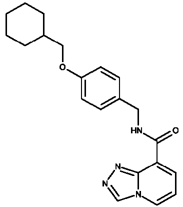 | 364.45 | 365.20 | | A | E |
| 50 | 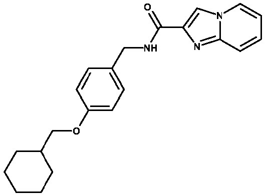 | 363.46 | 364.20 | | A | E |
| 51 | 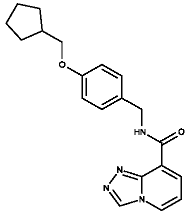 | 350.42 | 351.20 | | A | E |
| 52 | 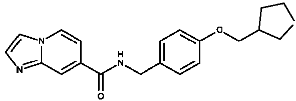 | 349.43 | 350.20 | | A | E |
| 53 | 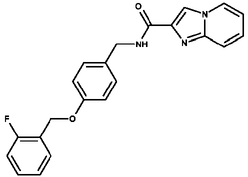 | 375.403 | 376.25 | | C | F |

### Pharmacology

### Cell lines

hNav1.7, hNav1.2, hNav1.3, hNav1.4, hNav1.5, hNav1.6 were stably expressed in human embryonic kidney cells and kept under constant antibiotic selection. For in vitro assays, cells were maintained in appropriate growth medium at 37 ºC and 5% CO₂ in a humidified incubator.

### Electrophysiology

Nav1.x expressing cells plated in T-25 flasks for 2-3 days prior to use and grown to 70-85% confluence were briefly trypsinized to obtain a single cell suspension of ~2-3 × 10⁶ cells/ml. All electrophysiological recordings were performed at ambient temperature (21-23 ºC) using automated patch clamp (Patchliner, Nanion Technologies GmbH) in the whole-cell configuration using the following solutions: Internal solution contained (in mM): CsCI (50), NaCl (10), CsF (60), EGTA (20), HEPES (10), pH 7.2 KOH, 285 mOsmol.Kg⁻¹. External (bath) solution contained (in mM): NaCl (140), KCl (4), MgCl₂ (1), CaCl₂ (2) D-Glucose monohydrate (5), HEPES (10), pH 7.4 NaOH, 298 mOsmol.kg⁻¹.

The following voltage protocols were used to assess different Nav1.x channel states:

### Resting state:

Cells were held at -120 mV and activated by 6 × 20 ms depolarising voltage pulses to -10 mV at a frequency of 0.1 Hz. Recordings were made initially in bath solution and 2-3 minutes after compound addition at increasing concentrations and steady state values were calculated from the 6^{th} pulse (P6). % resting state inhibition was calculated by (1-(P6drug/P6control))*100%.

### Inactivated state:

For each Nav1.x channel, a steady state inactivation curve was obtained in order to calculate the voltage resulting in 50% channel inactivation (V_{0.5inact}). A 2 pulse protocol was then employed in which cells held at a voltage corresponding to 20 mV more negative to V_{0.5 inact} were depolarised to -10 mV for 20 ms before returning to the previous holding voltage. This test pulse constituted P1 and represents 100% available (non- inactivated) Nav1.x channels. The same cell was then depolarised for 8 s to a voltage corresponding to 7 mV more positive than V_{0.5inact} in order to cause channel inactivation. Following a brief (2 ms) hyperpolarising voltage step to -120 mV (to reset the activation gate), cells were again subjected to a depolarising test pulse (P2) to -10 mV for 20 ms before returning to the holding potential. Each sequence was repeated 6 × at 30 s intervals. Using this protocol, control P2 current amplitudes (representing the % channels available following 8 s inactivation) was ~ 50% of control P1 current amplitude. 6 repetitions of this recording sequence were made in the control bath solution and then again 2 minutes after compound addition and thereafter at increasing compound concentrations. % inhibition was calculated by (1-(P2drug at repetition 6/P2control at repetition 6))*100. Data were either presented as % block by a single concentration of drug or an estimated eIC₅₀ determined from 2-3 drug concentrations which was determined by fitting data with a sigmoidal dose response curve (variable slope) using Graph Pad Prism software (Version 6.0).

### Compound preparation

All compounds were dissolved in DMSO to 20 mM initially. Subsequent dilutions in DMSO were made to achieve 200 × final bath concentrations. DMSO compound stocks were finally diluted 1/200 in bath solution just prior to assay resulting in a final DMSO concentration of 0.5% which was not found to affect control current amplitude.

**Table 2.**

| The below Compounds of Numbers 1, 3 to 6, 8, 9, 11, 12, 16, 17, 21 to 23, 32 to 36 and 41 to43 are according to the invention if used for treating or preventing pain disorders. | | | | |
|---|---|---|---|---|
| Number | Structure | hNav1.7 SIS (% inh) | Assay Conc. (uM) | hNav1.7 SIS (IC50 uM) |
| 1 | 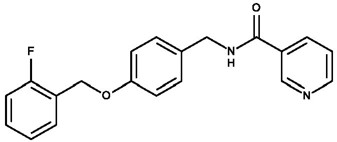 | 41 | 10 | |
| 2 | 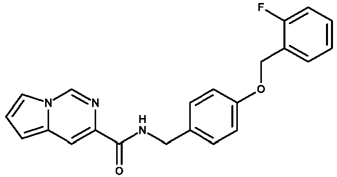 | 48 | 10 | |
| 3 | 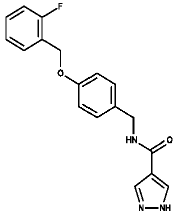 | 34 | 10 | |
| 4 | 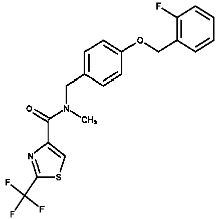 | 36 | 10 | |
| 5 | 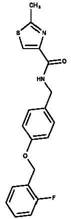 | 39 | 10 | |
| 6 | 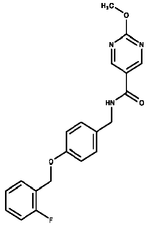 | 47 | 10 | |
| 7 | 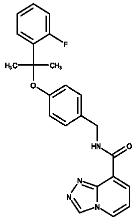 | 35 | 10 | |
| 8 | 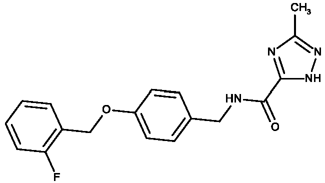 | 44 | 10 | |
| 9 | 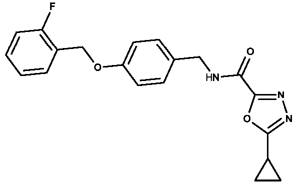 | 35 | 10 | |
| 10 | 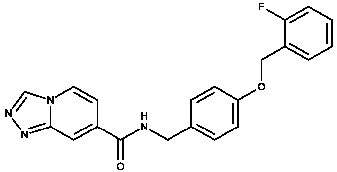 | 30 | 10 | |
| 11 | 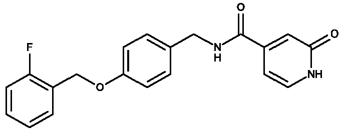 | 34 | 10 | |
| 12 | 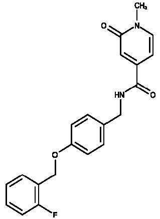 | 35 | 10 | |
| 13 | 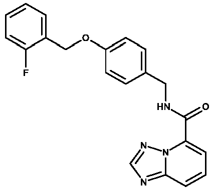 | 42 | 10 | |
| 14 | 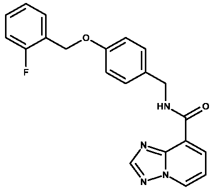 | 49 | 10 | |
| 15 | 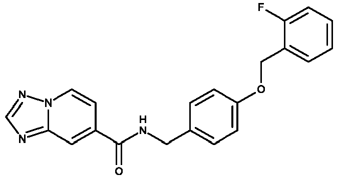 | 51 | 10 | |
| 16 | 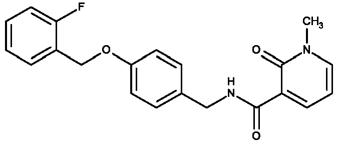 | 32 | 10 | |
| 17 | 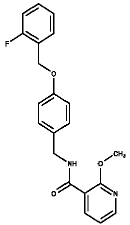 | 32 | 10 | |
| 18 | 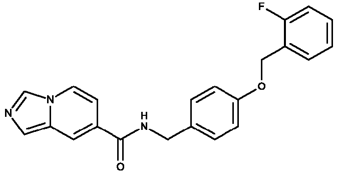 | 50 | 10 | |
| 19 | 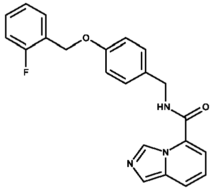 | 35 | 10 | |
| 20 | 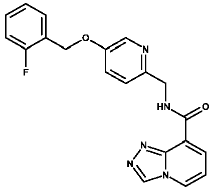 | 36 | 10 | |
| 21 | 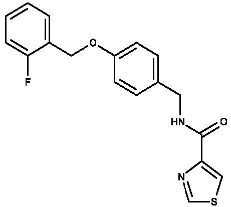 | 52 | 10 | |
| 22 | 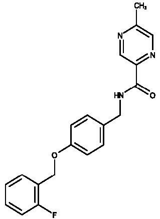 | 59 | 10 | |
| 23 | 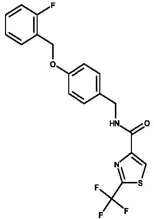 | 61 | 3 | 1.9 |
| 24 | 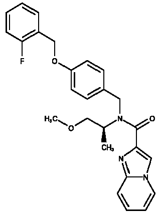 | 63 | 10 | 7.2 |
| 25 | 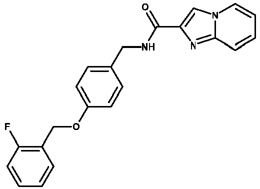 | 71 | 10 | 5.7 |
| 26 | 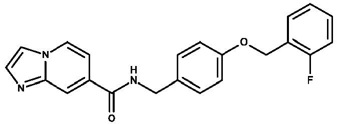 | | | 5.2 |
| 27 | 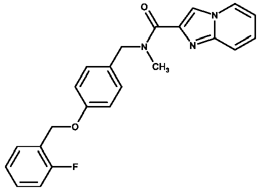 | 92 | 10 | 4.3 |
| 28 | 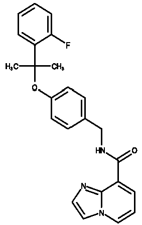 | | | 2.6 |
| 29 | 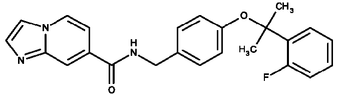 | | | 6.5 |
| 30 | 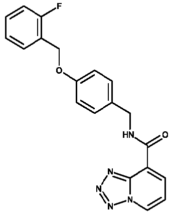 | | | 7.5 |
| 31 | 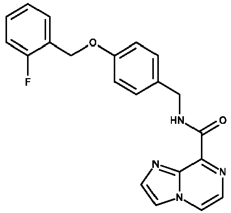 | | | 3.2 |
| 32 | 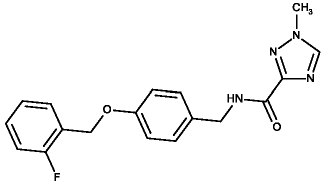 | | | 5.9 |
| 33 | 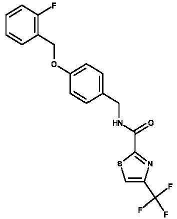 | | | 7.5 |
| 34 | 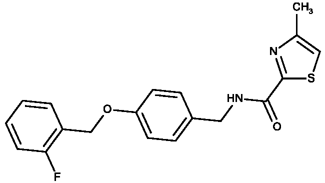 | | | 9.4 |
| 35 | 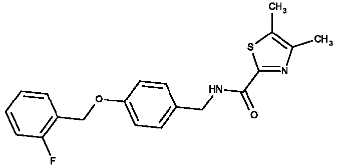 | | | 5.5 |
| 36 | 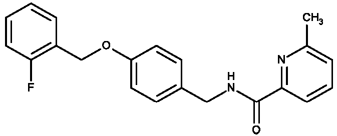 | | | 5.6 |
| 37 | 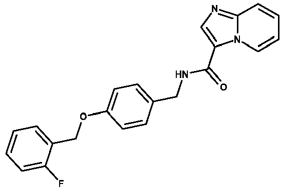 | | | 8.9 |
| 38 | 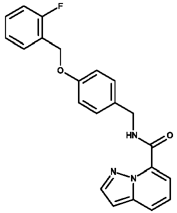 | | | 3.2 |
| 39 | 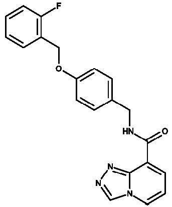 | | | 4.1 |
| 40 | 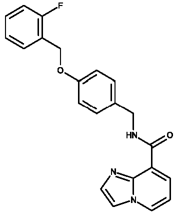 | | | 3.5 |
| 41 | 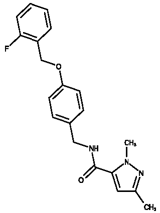 | | | 8.9 |
| 42 | 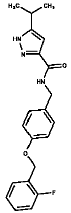 | | | 6.2 |
| 43 | 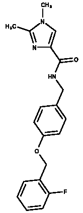 | | | 8.6 |
| 44 | 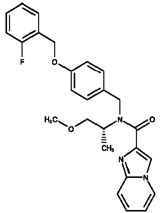 | | | 6.3 |
| 45 | 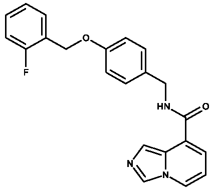 | | | 7.6 |
| 46 | 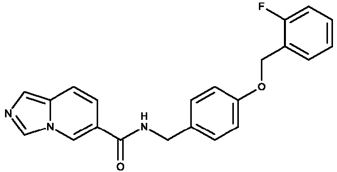 | 34 | 10 | |
| 47 | 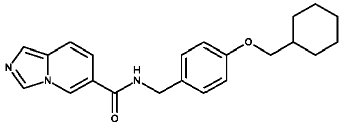 | | | 3.6 |
| 48 | 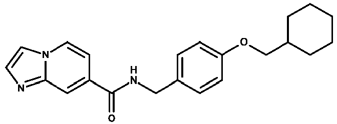 | | | 5.7 |
| 49 | 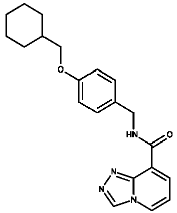 | | | 2.5 |
| 50 | 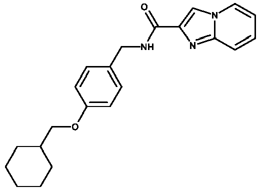 | | | 3.2 |
| 51 | 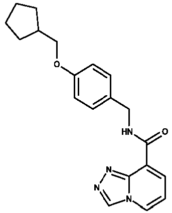 | | | 0.98 |
| 52 | 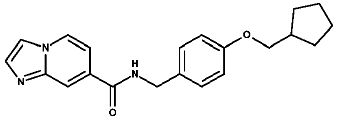 | 45 | 10 | |
| 53 | 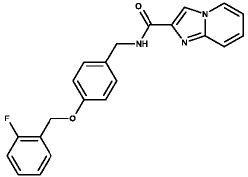 | 56 | 10 | |

## Claims

1. A compound of the formula (I) 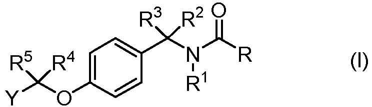
or salts, stereoisomers, or solvates thereof,
wherein
R is optionally substituted 7-12 membered heteroaryl with 2 or more N atoms;
R¹ is H or C₁-C₄ alkyl;
R², R³, R⁴ and R⁵ are independently H or C₁-C₄ alkyl, or R² and R³, or R⁴ and R⁵ together form a cycloalkyl ring;
Y is selected from 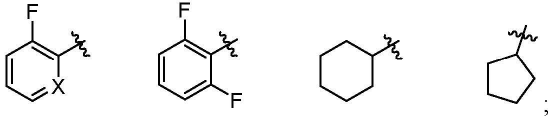
and
Xis CH or N,
wherein the optional substituents are selected from the group consisting of halogen, C₁₋₆ alkyl, C₁₋₆ alkoxy, C₂₋₆ alkenyl, C₂₋₆ alkynyl, C₁₋₆ haloalkyl, C₁₋₆ haloalkoxy, C₁₋₆ alkoxy(C₁₋₆)alkyl, C₃₋₆ cycloalkyl, -OH, phenyl, benzyl, phenoxy, benzyloxy, benzoyl, -NH₂, -NHC₁₋₄ alkyl, -N(C₁₋₄ alkyl)₂, -CN, -NO₂, mercapto, -P=O(OH)(NH₂), - S(O)₂NH₂, -S(O)₂NHC₁₋₄ alkyl, -S(O)₂N(C₁₋₄ alkyl)₂, C₁₋₆ alkylcarbonyl, C₁₋₆ alkoxycarbonyl, CO₂H, -S(O)R"' (where R‴ is C₁₋₆ alkyl or cycloalkyl) and -S(O)₂R‴ (where R‴ is C₁₋₆ alkyl, cycloalkyl or OH), and
wherein the "cycloalkyl ring" is a ring selected from cyclopropyl, cyclobutyl, cyclopentyl and cyclohexyl.

2. The compound of the formula (I) according to claim 1 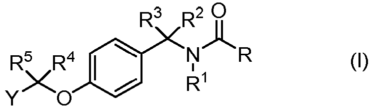
or salts, stereoisomers, or solvates thereof,
wherein
R is optionally substituted 7-12 membered heteroaryl with 2 nitrogen atoms;
R¹ is H or C₁-C₄ alkyl;
R², R³, R⁴ and R⁵ are independently H or C₁-C₄ alkyl, or R² and R³, or R⁴ and R⁵ together form a cycloalkyl ring;
Y is selected from 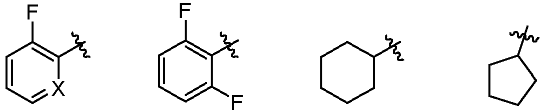 ; and
X is CH or N.

3. The compound according to claim 1 or 2, wherein R¹ is H.

4. The compound according to any one of claims 1 to 3, wherein R², R³, R⁴ and R⁵ are H.

5. The compound according to any one of claims 1 to 4, wherein Y is

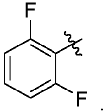

6. The compound according to any one of claims 1 to 4, wherein Y is

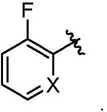

7. The compound according to claim 6, wherein X is CH.

8. The compound according to any one of claims 1 to 7, wherein R is selected from

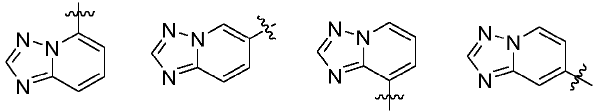

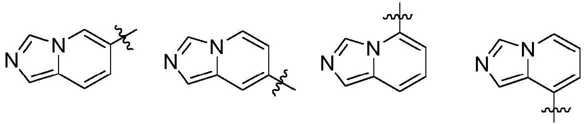

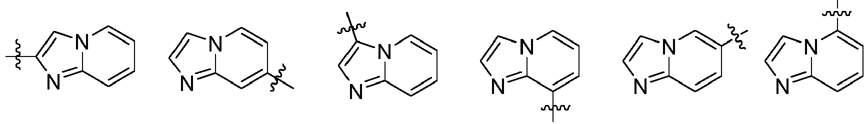

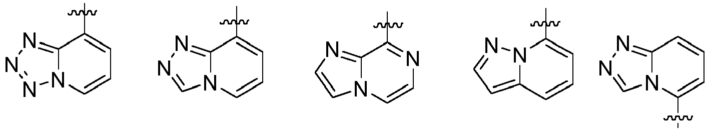

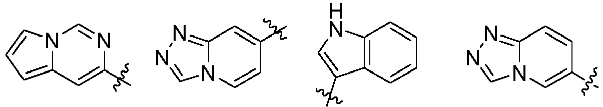

9. A pharmaceutical composition comprising a compound according to any one of claims 1 to 8, together with a diluent or carrier adjuvant.

10. A compound of formula (I) 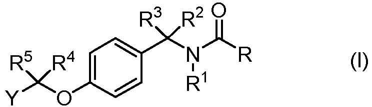
or salts, stereoisomers, or solvates thereof, for use in treating or preventing pain disorders,
wherein
R is optionally substituted aryl, optionally substituted heterocycyl or optionally substituted heteroaryl;
R¹ is H or optionally substituted C₁-C₄ alkyl;
R², R³, R⁴ and R⁵ are independently H or C₁-C₄ alkyl or R² and R³, or R⁴ and R⁵ together form a cycloalkyl ring;
Y is selected from 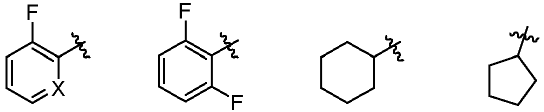 ; and
Xis CH or N,
wherein the optional substituents are selected from the group consisting of halogen, C₁₋₆ alkyl, C₁₋₆ alkoxy, C₂₋₆ alkenyl, C₂₋₆ alkynyl, C₁₋₆ haloalkyl, C₁₋₆ haloalkoxy, C₁₋₆ alkoxy(C₁₋₆)alkyl, C₃₋₆ cycloalkyl, -OH, phenyl, benzyl, phenoxy, benzyloxy, benzoyl, -NH₂, -NHC₁₋₄ alkyl, -N(C₁₋₄ alkyl)₂, -CN, -NO₂, mercapto, -P=O(OH)(NH₂), - S(O)₂NH₂, -S(O)₂NHC₁₋₄ alkyl, -S(O)₂N(C₁₋₄ alkyl)₂, C₁₋₆ alkylcarbonyl, C₁₋₆ alkoxycarbonyl, CO₂H, -S(O)R"' (where R‴ is C₁₋₆ alkyl or cycloalkyl) and -S(O)₂R‴ (where R‴ is C₁₋₆ alkyl, cycloalkyl or OH), and
wherein the "cycloalkyl ring" is a ring selected from cyclopropyl, cyclobutyl, cyclopentyl and cyclohexyl;
"aryl" is selected from phenyl, biphenyl, napthyl, tetrahydronaphthyl, indenyl, azulenyl, fluorenyl and anthracenyl;
"heterocyclyl" is a monovalent saturated or unsaturated group with 1 to 8 carbon atoms and from 1 to 4 heteroatoms selected from N, S and O; and
"heteroaryl" is a monovalent aromatic carbocyclic group with 2 to 10 carbon atoms and 1 to 4 heteroatoms selected from O, N and S.

11. The compound of the formula (I) for use according to claim 10, 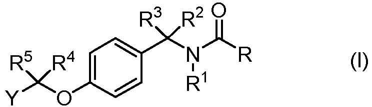
or salts, stereoisomers, or solvates thereof,
wherein
R is optionally substituted 7-12 membered heteroaryl with 2 or more N atoms;
R¹ is H or C₁-C₄ alkyl;
R², R³, R⁴ and R⁵ are independently H or C₁-C₄ alkyl, or R² and R³, or R⁴ and R⁵ together form a cycloalkyl ring;
Y is selected from 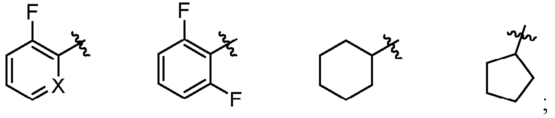 and
X is CH or N.

12. The compound of the formula (I) for use according to claim 10 or 11, 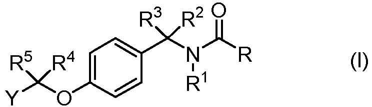
or salts, stereoisomers, or solvates thereof,
wherein
R is optionally substituted 7-12 membered heteroaryl with 2 nitrogen atoms;
R¹ is H or C₁-C₄ alkyl;
R², R³, R⁴ and R⁵ are independently H or C₁-C₄ alkyl, or R² and R³, or R⁴ and R⁵ together form a cycloalkyl ring;
Y is selected from 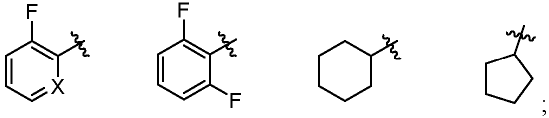 and
X is CH or N.

13. The compound for use according to any one of claims 10 to 12, wherein Y is

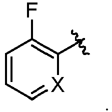

14. A compound for use according to claim 13, wherein X is CH.

15. The compound for use according to any one of claims 10 to 14, wherein the pain is selected from the group consisting of chronic pain, neuropathic pain, inflammatory pain, and cancer pain.

## Patentansprüche

1. Eine Verbindung der Formel (I) 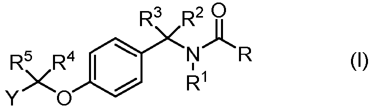
oder Salze, Stereoisomere oder Solvate davon,
wobei
R für gegebenenfalls substituiertes 7-12-gliedriges Heteroaryl mit 2 oder mehr N-Atomen steht;
R₁ für H oder C₁-C₄-Alkyl steht;
R², R³, R⁴ und R⁵ unabhängig für H oder C₁-C₄-Alkyl stehen, oder R² und R³, oder R⁴ und R⁵ zusammen einen Cycloalkylring bilden;
Y ausgewählt ist aus 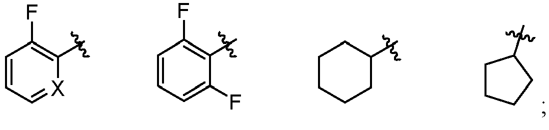 und
X für CH oder N steht,
wobei die gegebenenfalls vorhandenen Substituenten ausgewählt sind aus der Gruppe bestehend aus Halogen, C₁₋₆-Alkyl, C₁₋₆-Alkoxy, C₂₋₆-Alkenyl, C₂₋₆-Alkinyl, C₁₋₆-Halogenalkyl, C₁₋₆-Halogenalkoxy, C₁₋₆-Alkoxy(C₁₋₆)alkyl, C₃₋₆-Cycloalkyl, -OH, Phenyl, Benzyl, Phenoxy, Benzyloxy, Benzoyl, -NH₂, -NHC₁₋₄-Alkyl, -N(C₁₋₄-Alkyl)₂, -CN, -NO₂, Mercapto, -P=O(OH)(NH₂), -S(O)₂NH₂, -S(O)₂NHC₁₋₄-Alkyl, -S(O)₂N(C₁₋₄-Alkyl)₂, C₁₋₆-Alkylcarbonyl, C₁₋₆-Alkoxycarbonyl, CO₂H, -S(O)R"' (wobei R‴ für C₁₋₆-Alkyl oder Cycloalkyl steht) und -S(O)₂R‴ (wobei R‴ für C₁₋₆-Alkyl, Cycloalkyl oder OH steht), und
wobei der "Cycloalkylring" ein Ring ist, welcher ausgewählt ist aus Cyclopropyl, Cyclobutyl, Cyclopentyl und Cyclohexyl.

2. Die Verbindung der Formel (I) gemäß Anspruch 1 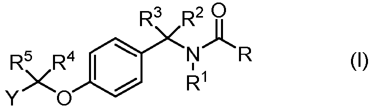
oder Salze, Stereoisomere oder Solvate davon,
wobei
R für gegebenenfalls substituiertes 7-12-gliedriges Heteroaryl mit 2 Stickstoffatomen steht;
R₁ für H oder C₁-C₄-Alkyl steht;
R², R³, R⁴ und R⁵ unabhängig für H oder C₁-C₄-Alkyl stehen, oder R² und R³, oder R⁴ und R⁵ zusammen einen Cycloalkylring bilden;
Y ausgewählt ist aus 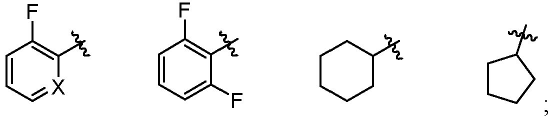 und
X für CH oder N steht.

3. Die Verbindung gemäß Anspruch 1 oder 2, wobei R¹ für H steht.

4. Die Verbindung gemäß einem der Ansprüche 1 bis 3, wobei R², R³, R⁴ und R⁵ für H stehen.

5. Die Verbindung gemäß einem der Ansprüche 1 bis 4, wobei Y für 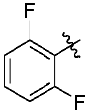 steht.

6. Die Verbindung gemäß einem der Ansprüche 1 bis 4, wobei Y für 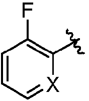 steht.

7. Die Verbindung gemäß Anspruch 6, wobei X für CH steht.

8. Die Verbindung gemäß einem der Ansprüche 1 bis 7, wobei R ausgewählt ist aus

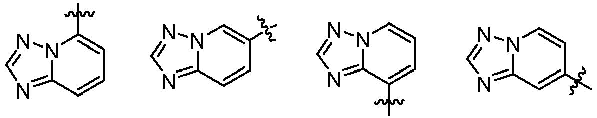

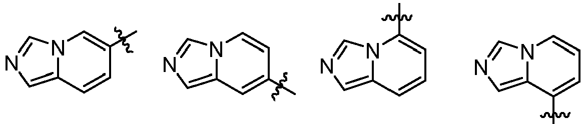

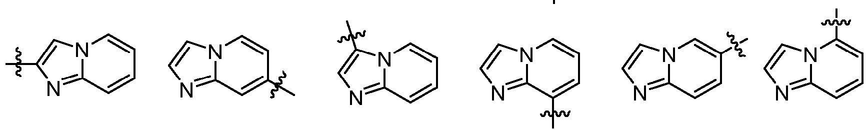

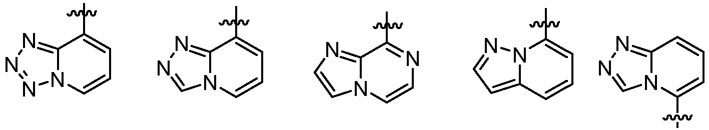

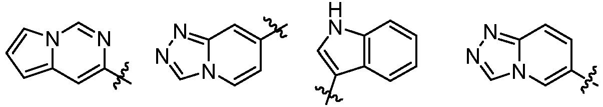

9. Ein Arzneimittel, umfassend eine Verbindung gemäß einem der Ansprüche 1 bis 8 zusammen mit einem Verdünnungsmittel oder einem Träger-Hilfsstoff.

10. Eine Verbindung der Formel (I) 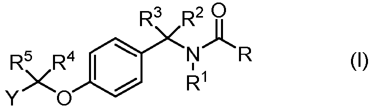
oder Salze, Stereoisomere oder Solvate davon, zur Verwendung bei der Behandlung oder Vorbeugung von Schmerzerkrankungen,
wobei
R für gegebenenfalls substituiertes Aryl, gegebenenfalls substituiertes Heterocyclyl oder gegebenenfalls substituiertes Heteroaryl steht;
R¹ für H oder gegebenenfalls substitutiertes C₁-C₄-Alkyl steht;
R², R³, R⁴ und R⁵ unabhängig für H oder C₁-C₄-Alkyl stehen, oder R² und R³, oder R⁴ und R⁵ zusammen einen Cycloalkylring bilden;
Y ausgewählt ist aus 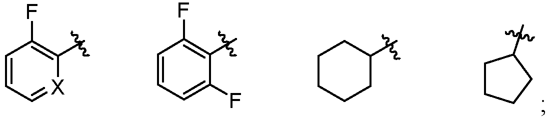 und
X für CH oder N steht,
wobei die gegebenenfalls vorhandenen Substituenten ausgewählt sind aus der Gruppe bestehend aus Halogen, C₁₋₆-Alkyl, C₁₋₆-Alkoxy, C₂₋₆-Alkenyl, C₂₋₆-Alkinyl, C₁₋₆-Halogenalkyl, C₁₋₆-Halogenalkoxy, C₁₋₆-Alkoxy(C₁₋₆)alkyl, C₃₋₆-Cycloalkyl, -OH, Phenyl, Benzyl, Phenoxy, Benzyloxy, Benzoyl, -NH₂, -NHC₁₋₄-Alkyl, -N(C₁₋₄-Alkyl)₂, -CN, -NO₂, Mercapto, -P=O(OH)(NH₂), -S(O)₂NH₂, -S(O)₂NHC₁₋₄-Alkyl, -S(O)₂N(C₁₋₄-Alkyl)₂, C₁₋₆-Alkylcarbonyl, C₁₋₆-Alkoxycarbonyl, CO₂H, -S(O)R"' (wobei R‴ für C₁₋₆-Alkyl oder Cycloalkyl steht) und -S(O)₂R‴ (wobei R‴ für C₁₋₆-Alkyl, Cycloalkyl oder OH steht), und
wobei der "Cycloalkylring" ein Ring ist, welcher ausgewählt ist aus Cyclopropyl, Cyclobutyl, Cyclopentyl und Cyclohexyl;
"Aryl" ausgewählt ist aus Phenyl, Biphenyl, Naphthyl, Tetrahydronaphthyl, Indenyl, Azulenyl, Fluorenyl und Anthracenyl;
"Heterocyclyl" eine einwertige gesättigte oder ungesättigte Gruppe mit 1 bis 8 Kohlenstoffatomen und mit 1 bis 4 Heteroatomen, ausgewählt aus N, S und O, ist; und
"Heteroaryl" eine einwertige aromatische carbocyclische Gruppe mit 2 bis 10 Kohlenstoffatomen und 1 bis 4 Heteroatomen, ausgewählt aus O, N und S, ist.

11. Die Verbindung der Formel (I) zur Verwendung gemäß Anspruch 10, 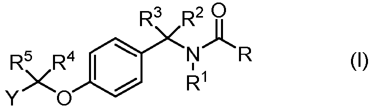
oder Salze, Stereoisomere oder Solvate davon,
wobei
R für gegebenenfalls substituiertes 7-12-gliedriges Heteroaryl mit 2 oder mehr N-Atomen steht;
R₁ für H oder C₁-C₄-Alkyl steht;
R², R³, R⁴ und R⁵ unabhängig für H oder C₁-C₄-Alkyl stehen, oder R² und R³, oder R⁴ und R⁵ zusammen einen Cycloalkylring bilden;
Y ausgewählt ist aus 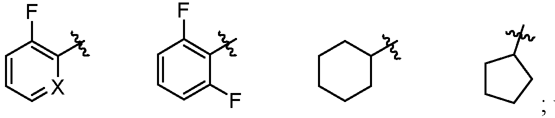 und
X für CH oder N steht.

12. Die Verbindung der Formel (I) zur Verwendung gemäß Anspruch 10 oder 11, 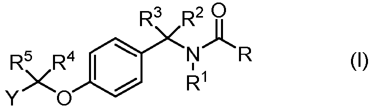
oder Salze, Stereoisomere oder Solvate davon,
wobei
R für gegebenenfalls substituiertes 7-12-gliedriges Heteroaryl mit 2 Stickstoffatomen steht;
R₁ für H oder C₁-C₄-Alkyl steht;
R², R³, R⁴ und R⁵ unabhängig für H oder C₁-C₄-Alkyl stehen, oder R² und R³, oder R⁴ und R⁵ zusammen einen Cycloalkylring bilden;
Y ausgewählt ist aus 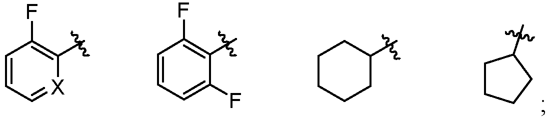 und
X für CH oder N steht.

13. Die Verbindung zur Verwendung gemäß einem der Ansprüche 10 bis 12, wobei Y für 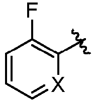 steht.

14. Eine Verbindung zur Verwendung gemäß Anspruch 13, wobei X für CH steht.

15. Die Verbindung zur Verwendung gemäß einem der Ansprüche 10 bis 14, wobei der Schmerz ausgewählt ist aus der Gruppe bestehend aus chronischem Schmerz, neuropathischem Schmerz, entzündlichem Schmerz, und Krebsschmerz.

## Revendications

1. Composé de formule (I) 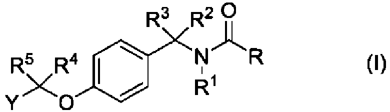
ou sels, stéréoisomères, ou solvates de celui-ci,
dans lequel
R est un hétéroaryle de 7 à 12 chaînons, ayant 2 ou plus d' atomes N, éventuellement substitué ;
R¹ est H ou un alkyle en C₁ à C₄ ;
R², R³, R⁴ et R⁵ sont indépendamment H ou un alkyle en C₁ à C₄, ou R² et R³, ou R⁴ et R⁵, forment ensemble un cycle cycloalkyle ;
Y est choisi parmi 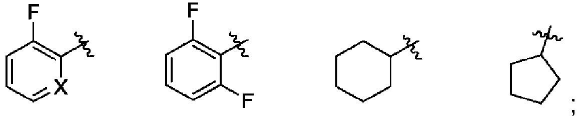
et X est CH ou N,
dans lequel les substituants optionnels sont choisis dans le groupe constitué par un halogène, alkyle en C₁ à C₆, alcoxy en C₁ à C₆, alcényle en C₂ à C₆, alcynyle en C₂ à C₆, halogénoalkyle en C₁ à C₆, halogénoalcoxy en C₁ à C₆, (alcoxy en C₁ à C₆)alkyle en C₁ à C₆, cycloalkyle en C₃ à C₆, -OH, phényle, benzyle, phénoxy, benzyloxy, benzoyle, -NH₂, -NH-alkyle en C₁ à C₄, -N(alkyle en C₁ à C₄)₂, -CN, -NO₂, mercapto, -P=O(OH)(NH₂), -S(O)₂NH₂, -S(O)₂NH-alkyle en C₁ à C₄, -S(O)₂N(alkyle en C₁ à C₄)₂, (alkyle en C₁ à C₆)carbonyle, (alcoxy en C₁ à C₆)carbonyle, CO₂H, -S(O)R‴ (où R‴ est un cycloalkyle ou alkyle en C₁ à C₆) et -S(O)₂R‴ (où R‴ est un cycloalkyle ou alkyle en C₁ à C₆ ou OH), et
dans lequel le "cycle cycloalkyle" est un cycle choisi parmi un cyclopropyle, un cyclobutyle, un cyclopentyle et un cyclohexyle.

2. Composé de formule (I) selon la revendication 1 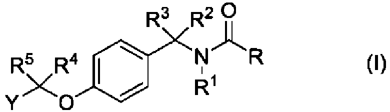
ou sels, stéréoisomères, ou solvates de celui-ci,
dans lequel
R est un hétéroaryle de 7 à 12 chaînons ayant 2 atomes d'azote, éventuellement substitué, ;
R¹ est H ou un alkyle en C₁ à C₄ ;
R², R³, R⁴ et R⁵ sont indépendamment H ou un alkyle en C₁ à C₄, ou R² et R³, ou R⁴ et R⁵, forment ensemble un cycle cycloalkyle ;
Y est choisi parmi 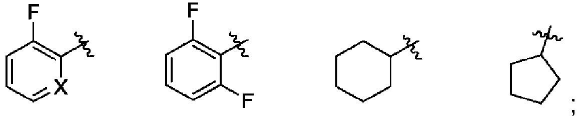
et X est CH ou N.

3. Composé selon la revendication 1 ou 2, dans lequel R¹ est H.

4. Composé selon l'une quelconque des revendications 1 à 3, dans lequel R², R³, R⁴ et R⁵ sont H.

5. Composé selon l'une quelconque des revendications 1 à 4, dans lequel Y est

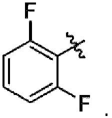

6. Composé selon l'une quelconque des revendications 1 à 4, dans lequel Y est

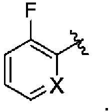

7. Composé selon la revendication 6, dans lequel X est CH.

8. Composé selon l'une quelconque des revendications 1 à 7, dans lequel R est choisi parmi

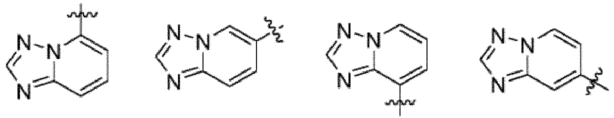

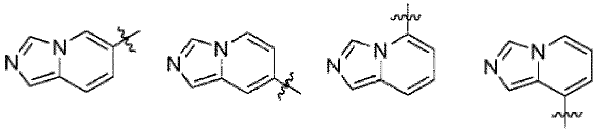

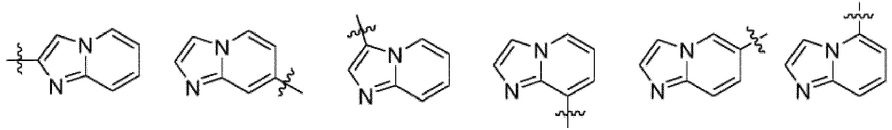

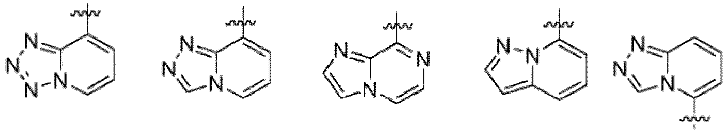

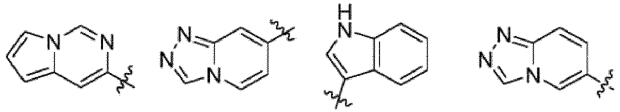

9. Composition pharmaceutique comprenant un composé selon l'une quelconque des revendications 1 à 8 conjointement avec un diluant ou un adjuvant porteur.

10. Composé de formule (I) 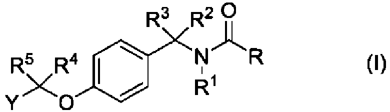
ou sels, stéréoisomères, ou solvates de celui-ci, pour une utilisation dans le traitement ou la prévention de troubles douloureux,
dans lequel
R est un aryle éventuellement substitué, un hétérocyclyle éventuellement substitué ou un hétéroaryle éventuellement substitué ;
R¹ est H ou un alkyle en C₁ à C₄ éventuellement substitué ;
R², R³, R⁴ et R⁵ sont indépendamment H ou un alkyle en C₁ à C₄, ou bien R² et R³, ou bien R⁴ et R⁵, forment ensemble un cycle cycloalkyle ;
Y est choisi parmi 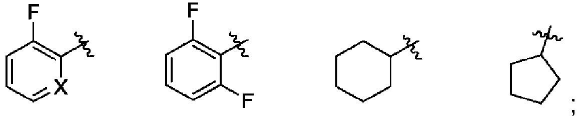
et X est CH ou N,
dans lequel les substituants optionnels sont choisis dans le groupe constitué par un halogène, alkyle en C₁ à C₆, alcoxy en C₁ à C₆, alcényle en C₂ à C₆, alcynyle en C₂ à C₆, halogénoalkyle en C₁ à C₆, halogénoalcoxy en C₁ à C₆, (alcoxy en C₁ à C₆)alkyle en C₁ à C₆, cycloalkyle en C₃ à C₆, -OH, phényle, benzyle, phénoxy, benzyloxy, benzoyle, -NH₂, -NH-alkyle en C₁ à C₄, -N(alkyle en C₁ à C₄)₂, -CN, -NO₂, mercapto, -P=O(OH)(NH₂), -S(O)₂NH₂, -S(O)₂NH-alkyle en C₁ à C₄, -S(O)₂N(alkyle en C₁ à C₄)₂, (alkyle en C₁ à C₆)carbonyle, (alcoxy en C₁ à C₆)carbonyle, CO₂H, -S(O)R‴ (où R‴ est un cycloalkyle ou alkyle en C₁ à C₆) et-S(O)₂R‴ (où R‴ est un cycloalkyle ou alkyle en C₁ à C₆ ou OH), et
dans lequel le "cycle cycloalkyle" est un cycle choisi parmi un cyclopropyle, un cyclobutyle, un cyclopentyle et un cyclohexyle ;
"aryle" est choisi parmi un phényle, un biphényle, un naphtyle, un tétrahydronaphtyle, un indényle, un azulényle, un fluorényle et un anthracényle ;
"hétérocyclyle" est un groupe monovalent saturé ou insaturé ayant 1 à 8 atomes de carbone et 1 à 4 hétéroatomes choisis parmi N, S et O ; et
"hétéroaryle" est un groupe aromatique carbocyclique monovalent ayant 2 à 10 atomes de carbone et 1 à 4 hétéroatomes choisis parmi O, N et S.

11. Composé de formule (I) pour une utilisation selon la revendication 10, 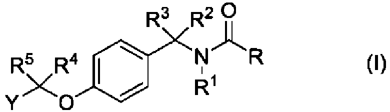 ou sels, stéréoisomères, ou solvates,
dans lequel
R est un hétéroaryle de 7 à 12 chaînons ayant 2 ou plus d'atomes N, éventuellement substitué ;
R¹ est H ou un alkyle en C₁ à C₄ ;
R², R³, R⁴ et R⁵ sont indépendamment H ou un alkyle en C₁ à C₄, ou bien R² et R³, ou bien R⁴ et R⁵, forment ensemble un cycle cycloalkyle ;
Y est choisi parmi 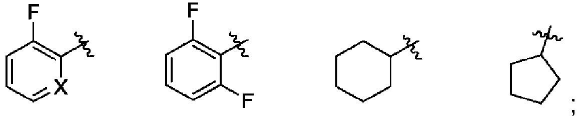
et X est CH ou N.

12. Composé de formule (I) pour une utilisation selon la revendication 10 ou 11, 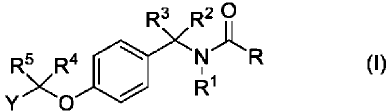
ou sels, stéréoisomères, ou solvates de celui-ci,
dans lequel
R est un hétéroaryle de 7 à 12 chaînons ayant 2 atomes d'azote, éventuellement substitué ;
R¹ est H ou un alkyle en C₁ à C₄ ;
R², R³, R⁴ et R⁵ sont indépendamment H ou un alkyle en C₁ à C₄, ou R² et R³, ou R⁴ et R⁵, forment ensemble un cycle cycloalkyle ;
Y est choisi parmi 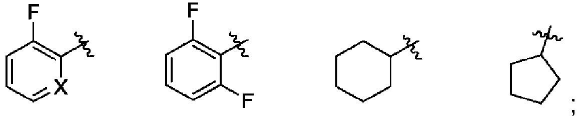
et X est CH ou N.

13. Composé pour une utilisation selon l'une quelconque des revendications 10 à 12, dans lequel Y est

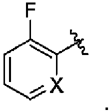

14. Composé pour une utilisation selon la revendication 13, dans lequel X est CH.

15. Composé pour une utilisation selon l'une quelconque des revendications 10 à 14, dans lequel la douleur est choisie dans le groupe constitué par une douleur chronique, une douleur neuropathique, une douleur inflammatoire, et une douleur liée à un cancer.
